# EUROPEAN PATENT APPLICATION

(11) **EP 1 417 974 A1**
(43) Date of publication of application: **12.05.2004**
(21) Application number: 02024881.1
(22) Date of filing: 08.11.2002
(51) Int. Cl.: A61K 41/00, A61K 47/48, A61P 35/00

(54) **Compositions and methods for treating cancer using cytotoxic CD44 antibody immunoconjugates and radiotherapy**

(71) Applicant: Boehringer Ingelheim International GmbH, 55216 Ingelheim am Rhein (DE)
(72) Inventor: The designation of the inventor has not yet been filed

(57) **Abstract**

The invention relates to the combined use of conjugates of antibodies with cytotoxic compounds and radiotherapy in cancer therapy, pharmaceutical compositions comprising such compounds, and methods of cancer treatment. In a preferred embodiment, the antibody is specific for the tumor-associated antigen CD44v6 and linked to a maytansinoid. The radiotherapy may be external beam radiotherapy or radioimmunotherapy.

## Description

The invention relates to the combined use of conjugates of antibodies with cytotoxic compounds and radiotherapy in cancer therapy, pharmaceutical compositions comprising such compounds, and methods of cancer treatment.

There have been numerous attempts to improve the efficacy of antineoplastic drugs by conjugating such drugs to antibodies against tumor-associated antigens in order to elevate local concentration of the drug by targeted delivery to the tumor. Many of these approaches have met limited success, and several reasons have been discussed in the literature to explain the failure. For anticancer drugs acting stoichometrically, like e.g. doxorubicin or methotrexate, relatively high intracellular concentrations are necessary to exert the required cytotoxicity. These concentrations are thought to be difficult to achieve with many antibody-drug conjugates because of (a) insufficient potency of many common anticancer drugs, (b) low cell surface concentration of antigen targets, (c) inefficient internalization of antigen-antibody complexes into the target cell, and (d) inefficient release of free drug from the conjugate inside the target cell (Chari et al., 1992).

Two of the aforementioned drawbacks, namely (a) and (d), have been adressed by the work of Chart and coworkers (Chari et a., 1992; Liu et al., 1996; U.S. Patent No. 5,208,020). They have developed antibody conjugates wherein the antibody is linked to a maytansinoid via a disulfide linkage. Maytansines belong to the class of Ansa macrolide antibiotics, which derive from *Nocardia sp..* The maytansine ansamitocin P-3, produced by bacterial fermentation, is used as a precursor molecule to manufacture maytansinoid DM1. Maytansine and derivatives act as anti-mitotic agents (inhibitors of tubulin polymerization), similar as vincristine, but with markedly higher potency than vincristine or other established chemotherapeutic agents (DM1 is toxic to cells *in vitro* at ~10⁻¹⁰ M concentration). In contrast to the high cytotoxicity of free maytansinoid, the antibody conjugate has a toxicity which is several orders of magnitude lower on antigen-negative cells compared to antigen-positive cells. The linkage by disulfide bonding has the advantage that these bonds are readily cleaved inside the target cells by intracellular glutathione, releasing highly toxic free drug. This approach has been applied to antibodies against tumor-associated antigens, for example the C242-DM1 conjugate (Liu et al., 1996; Lambert et al., 1998), and HuN901-DM1 (Chari et al., 2000). However, the application of these conjugates is restricted due to the limited expression of the respective target antigens. For example, the antigen recognized by N901 (CD56, N-CAM) is predominantly expressed by tumors of neuroendocrine origin, the expression of the C242 antigen (CanAg) is mostly limited to tumors derived from the GI tract.

There is, therefore, still the need to improve this approach by finding suitable tumor-associated antibodies with favorable antigen expression pattern, high and specific cell surface antigen concentration within the target tissue, and efficient internalization process transporting the antigen-complexed antibody conjugate into the cells.

CD44 is a protein which is expressed in several different isoforms on the surface of a wide variety of cell types. The smallest isoform, standard CD44 (CD44s), which is expressed by a variety of different cells, is thought to mediate cell attachment to extracellular matrix components and may transmit a co-stimulus in lymphocyte and monocyte activation. In contrast, expression of splice variants of CD44 which contain the domain v6 (CD44v6) in the extracellular region, is restricted to a subset of epithelia. The physiological role of CD44v6 is not yet fully understood.

CD44v6, as well as other variant exons (CD44v3, CD44v5, CD44v7/v8, CD44v10) has been shown to be a tumor-associated antigen with a favorable expression pattern in human tumors and normal tissues (Heider et al., 1995; Heider et al., 1996; Dall et al., 1996; Beham-Schmid et al., 1998; Tempfer et al., 1998; Wagner et al., 1998) and has been subject to antibody-based diagnostic and therapeutic approaches, in particular radioimmunotherapy (RIT) of tumors (Verel et al., 2002; Stromer et al., 2000, WO 95/33771, WO 97/21104).

However, a prerequisite for efficient killing of tumor cells by antibody maytansinoid conjugates is sufficient internalization of the target antigen. Only few data on the internalization of CD44 are available. Bazil et Horejsi reported that downregulation of CD44 on leukocytes upon stimulation with PMA is caused by shedding of the antigen rather than by internalization (Bazil et Horejsi, 1992). Shedding of CD44 is also supported by several reports on soluble CD44 in the serum of tumor patients and normal individuals (Sliutz et al., 1995; Guo et al., 1994; Martin et al., 1997). In a recent paper by Aguiar et al. the amount of internalized CD44 on matrix-intact chondrocytes was determined to be approximately 6% in 4 hours (Aguiar et al., 1999). Similar low levels of internalized CD44v6 on tumor cells were found in experiments performed by BIA. Taken together, these data suggest that CD44 receptors are more likely subject to shedding than to internalization, and thus CD44 specific antibodies are not to be regarded as suitable candidates for the maytansinoid conjugate approach. This has been supported by in vitro cell proliferation assays wherein Ab_{CD44v6}-DM1 showed only slightly elevated cytotoxicity against antigen-presenting cells as compared to cells lacking the antigen.

It now has been found that CD44 specific antibodies conjugated to highly cytotoxic drugs through a linker which is cleaved under intracellular conditions are very efficient tumor therapeutics in vivo. Thus, such compounds may be advantageously used in cancer therapy.

There is still the need for further improvements. For the antibody maytansinoid conjugates huN901-DM and C242-DM1, the combination of these conjugates with the taxanes paclitaxel or docetaxel has been suggested (WO 01/24763).

It has now been unexpectedly found that the combination of a conjugate consisting of a CD44 specific antibody and a cytotoxic agent with radiotherapy shows supraaditive effects. Such conjugates are highly effective radiosensitisers.

In particular, the present invention relates to the use of a compound of formula

**A(LB)**_{**n**} (Formula (I))

wherein
- **A**: is an antibody molecule which is specific for CD44;
- **L**: is a linker moiety;
- **B**: is a compound which is toxic to cells; and
- **n**: is a decimal number with n = 1 to 10
for the preparation of a pharmaceutical composition for the treatment of cancer, wherein said compound is used or is for use in combination with radiotherapy. In a further aspect, the present invention relates to a method of treatment of cancer, wherein an effective amount of a compound of Formula (I), as defined herein, is administered to a patient in need thereof in combination with radiotherapy.

The antibody molecule A has a binding specificity for CD44, preferably variant CD44, most preferably CD44v6.

The term "antibody molecule" shall encompass complete immunoglobulins as they are produced by lymphocytes and for example present in blood sera, monoclonal antibodies secreted by hybridoma cell lines, polypeptides produced by recombinant expression in host cells which have the binding specificity of immunoglobulins or monoclonal antibodies, and molecules which have been derived from such immunoglobulins, monoclonal antibodies, or polypeptides by further processing while retaining their binding specificity. In particular, the term "antibody molecule" includes complete immunoglobulins comprising two heavy chains and two light chains, fragments of such immunoglobulins like Fab, Fab', or F(ab)₂ fragments (Kreitman *et al.,* 1993), recombinantly produced polypeptides like chimeric, humanised or fully human antibodies (Breitling et Duebel, 1999; Shin *et al.,* 1989; Güssow *et* Seemann, 1991, Winter *et al.,* 1994, EP 0 239 400; EP 0 519 596; WO 90/07861 EP 0 368 684; EP 0 438 310; WO 92/07075; WO 92/22653; EP 0 680 040; EP 0 451 216), single chain antibodies (scFv, Johnson et Bird, 1991), multimeric antibodies (Kortt et al., 2001) like diabodies, triabodies, or tetrabodies, and the like. Today, antibodies may also be produced without immunising a laboratory animal, e.g. by phage display methods (Aujame et al., 1997; US 5,885,793; US 5,969,108; US 6,300,064; US 6,248,516, US 6,291,158). Fully human antibodies may be produced using transgenic mice carrying functional human Ig genes (EP 0 438 474 ; EP 0 463 151; EP 0 546 073). From the aforementioned literature references, the expert knows how to produce these types of antibody molecules, employing state of the art methods like automated peptide and nucleic acid synthesis, laboratory animal immunisation, hybridoma technologies, polymerase chain reaction (PCR), vector and expression technologies, host cell culture, and protein purification methods. In the following, the terms "antibody" and "antibody molecule" are used interchangeably. "Specific for CD44" shall mean that the antibody molecule has specific binding affinity for an epitope present in CD44. In a preferred embodiment, the antibody molecule of the invention has a binding specificity for the amino acid sequence coded by variant exon v6 of the human CD44 gene. The sequence of variant exon v6 as well as of the other variant exons is known in the art (Screaton *et al.,* 1992; Tölg *et al.,* 1993; Hofmann *et al.,* 1991). A preferred antibody molecule of the invention specifically binds to peptides or polypetides having or containing the amino acid sequence SEQ ID NO: 1 of the accompanying sequence listing, or an allelic variant of said sequence. Preferably, said antibody molecule has binding specificity for an epitope within said sequence. More preferably, the antibody molecule specifically binds to a peptide having the amino acid sequence SEQ ID NO: 2, even more preferably having the amino acid sequence SEQ ID NO: 3. Such antibody molecules may be easily produced with methods known in the art (WO 95/33771, WO 97/21104), e.g. by immunising laboratory animals with chemically synthesised peptides having the aforementioned sequences, e.g. bound to a hapten, or immunising with a recombinantly produced fusion protein including said sequences, and proceeding according to methods known in the art (Harlow 1988; Catty 1988; Koopman *et al.,* 1993; Heider *et al.,* 1993).

Preferably, an antibody molecule to be used for the present invention is the murine monoclonal antibody with the designation VFF-18 which is produced by a hybridoma cell line which has been deposited on 07 June 1994 under the accession number DSM ACC2174 with the DSM-Deutsche Sammlung für Mikroorganismen und Zellkulturen GmbH, Mascheroder Weg 1b, D-38124 Braunschweig, Deutschland/Germany. Also preferred are Fab, Fab', or F(ab)₂ fragments of said monoclonal antibody VFF-18. In another preferred embodiment, the antibody molecule is a humanised recombinant antibody, wherein the complementarity determining regions (CDR's) of VFF-18 have been grafted into the respective genes of human immunoglobulin heavy and light chains.

"Complementarity determining regions" of a monoclonal antibody are understood to be those amino acid sequences involved in specific antigen binding according to Kabat *et al.,* 1991, in connection with Chothia and Lesk, 1987.

In another preferred embodiment, appropriate framework residues of such a CDR-grafted antibody are reverted to murine residues to improve binding affinity. From methods pertinent to the art, the experts knows how to obtain the CDR's of VFF-18, starting with the aforementioned hybridoma with the accession number DSM ACC2174, to choose and obtain appropriate human immunoglobulin genes, to graft the CDR's into these genes, to modify selected framework residues, to express the CDR-grafted antibody in appropriate host cells, e.g. Chinese hamster ovary (CHO) cells, and to test the resulting recombinant antibodies for binding affinity and specificity (see e.g. literature references above). In another preferred embodiment of the invention, the antibody molecule is a recombinant antibody having the CDR's of the antibody VFF-18. Preferably, such a recombinant antibody is a humanised antibody and is a complete immunoglobulin consisting of two complete light and two complete heavy chains. In another preferred embodiment of the invention, the antibody molecule is a recombinant antibody having the same idiotype as the antibody VFF-18. In another preferred embodiment of the invention, the antibody moelcule is a recombinant antibody binding to the same epitope as the antibody VFF-18.

In a particular preferred embodiment, the antibody molecule A is an antibody comprising light chains having the amino acid sequence SEQ ID NO: 4, and heavy chains having the amino acid sequence SEQ ID NO: 6. This antibody is called BIWA 4. It is a humanised version of antibody VFF-18 mentioned above, having the complementary determining regions of the murine monoclonal antibody VFF-18 in a completely human framework, and human constant regions. It is therefore an antibody of very low immunogenicity in man, which is a favorable trait. However, as it has no murine framework residues to optimise antigen binding, it has a significanty lower antigen binding affinity as its parent antibody VFF-18, and therefore would not have been regarded as a good candidate for a therapeutic drug. Unexpectedly, it has been found that BIWA 4, despite its poor binding affinity, has a very favorable biodistribution and tumor uptake in vivo, making it superior to other humanised versions of VFF-18 with higher binding affinity. In a further preferred embodiment, the antibody molecule A is an antibody comprising light chains having the amino acid sequence SEQ ID NO: 8, and heavy chains having amino acid sequence SEQ ID NO: 6. This antibody is called BIWA 8 and has higher binding affinity than BIWA 4.

These antibodies may be produced as follows. Nucleic acid molecules coding for the light chain and the heavy chain may be synthesised chemically and enzymatically by standard methods. First, suitable oligonucleotides can be synthesized with methods known in the art (e.g. Gait MJ, 1984), which can be used to produce a synthetic gene. Methods to generate synthetic genes from oligonucleotides are known in the art (e.g. Stemmer et al. 1995; Ye et al. 1992; Hayden et Mandecki 1988; Frank et al. 1987). Preferably, the nucleic acid molecules encoding the light and heavy chains of BIWA 4 have the nucleotide sequences of SEQ ID NO: 5 and SEQ ID NO: 7, respectively. These sequences include sequences coding for leader peptides which are cleaved by the host cell (SEQ ID NO: 5: the first 60 nucleotides; SEQ ID NO: 7: the first 57 nucleotides). In a further embodiment, the nucleic acid molecules encoding the light and heavy chains of an antibody molecule according to the invention have the nucleotide sequences of SEQ ID NO: 9 and SEQ ID NO: 7, respectively. These nucleic acid molecules encoding the antibody heavy and light chains then may be cloned into an expression vector (either both chains in one vector molecule, or each chain into a separate vector molecule), which then is introduced into a host cell. Expression vectors suitable for immunoglobulin expression in prokaryotic or eukaryotic host cells and methods of introduction of vectors into host cells are well-known in the art. In general, the immunoglobulin gene therein is in functional connection with a suitable promoter, like for example a human cytomegalovirus (CMV) promoter, hamster ubiquitin promoter (WO 97/15664), or a simian virus SV40 promoter located upstream of the Ig gene. For termination of transcription, a suitable termination/polyadenylation site like that of the bovine growth hormone or SV40 may be employed. Furthermore, an enhancer sequence may be included, like the CMV or SV40 enhancer. Usually, the expression vector furthermore contains selection marker genes like the dihydrofolate reductase (DHFR), glutamine synthetase, adenosine deaminase, adenylate deaminase genes, or the neomycin, bleomycin, or puromycin resistance genes. A variety of expression vectors are commercially available from companies such as Stratagene, La Jolla, CA; Invitrogen, Carlsbad, CA; Promega, Madison, WI or BD Biosciences Clontech, Palo Alto, CA. For example, expression vectors pAD-CMV1 (NCBI GenBank Accession No. A32111) or pAD-CMV19 (NCBI GenBank Accession No. A32110) may be used for expression. The host cell preferably is a mamalian host cell, e.g. a COS, CHO, or BHK cell, more preferably a Chinese hamster ovary (CHO) cell, e.g. a CHO-DUKX (Urlaub and Chasin, 1980), CHO-DG44 (Urlaub et al., 1983), or CHO-K1 (ATCC CCL-61) cell. The host cell then is cultured in a suitable culture medium under conditions where the antibody is produced, and the antibody is then isolated from the culture according to standard procedures. Procedures for production of antibodies from recombinant DNA in host cells and respective expression vectors are well-known in the art (see e.g. WO 94/11523, WO 97/9351, EP 0 481 790, EP 0 669 986).

In order to link the antibody molecule **A** to the compound **B** which is toxic to cells, a linking moiety **L** is used. In the most simple case, the linking moiety **L** is a chemical bond, preferably a covalent bond which is cleaved under intracellular conditions. In one embodiment of the invention, the bond is between a sulfur atom present in the antibody molecule, e.g. in the side chain of a cystein residue, and another sulfur atom present in the toxic compound. In another embodiment, the linking moiety **L** consists of one or more atoms or chemical groups. Suitable linking groups are well known in the art and include disulfide groups, thioether groups, acid labile groups, photolabile groups, peptidase labile groups and esterase labile groups. Preferred are disulfide groups and thioether groups.

Conjugates of the antibody molecules of the invention and toxic compound can be formed using any techniques presently known or later developed. The toxic compound can be modified to yield a free amino group and then linked to the antibody molecule via an acid-labile linker, or a photolabile linker. The toxic compound can be condensed with a peptide and subsequently linked to an antibody molecule to produce a peptidase-labile linker. The toxic compound can be treated to yield a primary hydroxyl group, which can be succinylated and linked to an antibody molecule to produce a conjugate that can be cleaved by intracellular esterases to liberate free drug. Most preferably, the toxic compound is treated to create a free or protected thiol group, and then one or many disulfide or thiol-containing toxic compounds are covalently linked to the antibody molecule via disulfide bond(s).

For example, antibody molecules can be modified with crosslinking reagents such as N-succinimidyl 3-(2-pyridyldithio)propionate (SPDP), 4-succinimidyl-oxycarbonyl-α-methyl-α-(2-pyridyldithio)-toluene (SMPT), N-succinimidyl-3-(2-pyridyldithio)-butyrate (SDPB), N-succinimidyl-4-(2-pyridyldithio)pentanoate (SPP), N-succinimidyl-5-(2-pyridyldithio)pentanoate, 2-iminothiolane, or acetylsuccinic anhydride by known methods.

See, Carlsson et al, 1978; Blattler et al 1985; Lambert et al, 1983; Klotz et al, 1962; Liu et al, 1979; Blakey and Thorpe, 1988; Worrell et al, 1986. In a preferred embodiment, the linker moiety is a 5-thiopentanoate or 4-thiopentanoate derived from SPP. The antibody molecule containing free or protected thiol groups thus derived is then reacted with a disulfide- or thiol-containing toxic compound to produce conjugates. The conjugates can be purified by HPLC or by gel filtration.

"Toxic" is a compound that inhibits or prevents function of cells and/or causes cell destruction. Toxic compounds used for coupling may act either cytostatic or cytotoxic and lead to cell cycle arrest or cell death. These compounds may act at different stages during the cell cycle, e.g. by interference with nucleic acid synthesis, inactivation of nucleic acids, or by binding to tubulin.

In a preferred embodiment, the compound **B** present in **A(LB)**_{**n**} which is toxic to cells is a maytansinoid, i.e. a derivative of maytansine (CAS 35846538). In a preferred embodiment, it is a C-3 ester of maytansinol. Maytansinoids suitable for conjugating to antibodies for use in cancer therapy, including preparation of said maytansinoids and their linkage to antibody molecules, have been described by Chari and Coworkers (Chari et al., 1992; Liu et al., 1996; U.S. Patent No. 5,208,020). These maytansinoids may be used for the present invention. In a preferred embodiment, the toxic compound is N^{2'}-deacetyl- N^{2'}-(3-mercapto-1-oxopropyl)-Maytansine (CAS Number 139504-50-0), also referred to as DM1. Preferably, said maytansinoid is a maytansinol derivative linked to the antibody molecule via a disulfide bridge at the C-3 position of maytansinol. In a particularly preferred embodiment, the antibody/maytansinoid conjugate may be prepared from a maytansinoid of formula 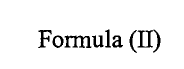 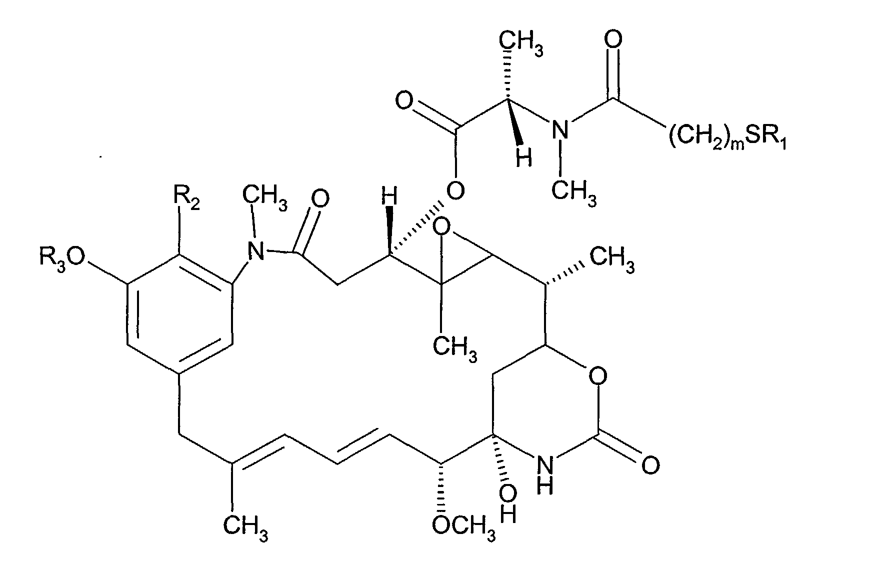 wherein
- R₁: represents H or SR₄, wherein R₄ represents methyl, ethyl, linear alkyl, branched alkyl, cyclic alkyl, simple or substituted aryl, or heterocyclic;
- R₂: represents Cl or H;
- R₃: represents H or CH₃; and
- m: represents 1, 2, or 3.

Preferably, R₁ is H, CH₃, or SCH₃, R₂ is Cl, R₃ is CH₃, and m = 2.

The compound with R₁ = H, R₂ = Cl, R₃ = CH₃, and m = 2 is designated DM1 in the literature.

In a preferred embodiment, the compound of the invention has the formula 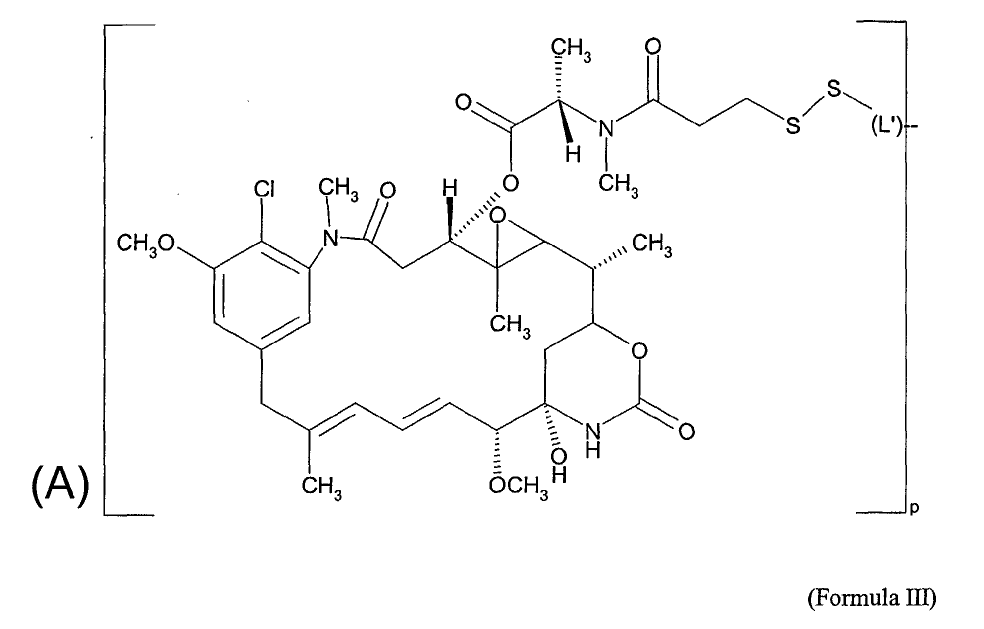 wherein
A is an antibody molecule which is specific for CD44, preferably specific for the variant exon v6, preferably specific for the amino acid sequence SEQ ID NO: 3;
(L') is an optional linker moiety
p is a decimal number with p = 1 to 10

Preferably, p is 3 to 4, more preferably about 3.5.

Methods for preparing such maytansinoids are known in the art (see in particular US 5,208,020, Example 1). Conveniently, in a first step the maytansinoid C-3 ester ansamitocin P3 may be produced by bacterial fermentation (US 4,356,265; US 4,450,234; WO 01/77360) of microorganisms belonging to the genus *Nocardia* or *Actinosynnema, e.g.* ATCC 31565, ATCC 31281. Ansamitocin P3 maybe extracted from the culture using organic solvents like ethyl acetate or toluene, and further purified by adsorption chromatography using e.g. silica gel. It may then be reduced to maytansinol using LiAlH₄ (US 4,360,462) or, as suggested more recently (WO 02/16368), LiAl(OMe)₃H or other LiAl or NaAl hydrids. The maytansinol may then be esterified at the C-3 position with N-methyl-L-alanine or N-methyl-L-cysteine derivatives to yield a disulfide-containing maytansinoid (US 5,208,020; US 5,416,064; US 6,333,410), for example using 1-1414-EP-Prio dicyclohexylcarbodiimide(DCC) and catalytic amounts of zinc chloride (US 4,137,230; US 4,260,609). In a preferred embodiment, the maytansinol is esterified with the compound N-methyl-N-(3-methyldithiopropanoyl)-L-alanine of formula 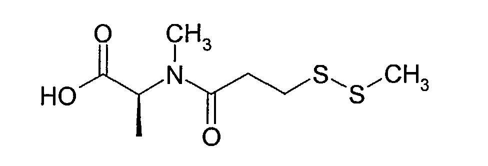 to yield the maytansinoid of Formula (IT) with with R₁ = SR₄, R₄ = CH₃, R₂ = Cl, R₃ = CH₃, and m = 2. Manufacture ofN-methyl-L-alanine or N-methyl-L-cysteine derivatives is disclosed for example in US 5,208,020 and WO 02/22554.

The free thiol group may then be released by cleavage of the disulfide bond with dithiothreitol (DTT), to yield e.g. DM1.

Upon intracellular cleavage, the free toxic compound is released. The free drug released from the compound **A(LB)**_{**n**} may have the formula **B-X,** wherein **X** is an atom or a chemical group, depending on the nature of the cleaving reaction. Preferably, **X** is a hydrogen atom, as for example when the linker moiety is just a covalent bond between two sulfur atoms, or a hydroxyl group. The cleavage site may also be within the linker moiety if the linker moiety is a chemical group, generating free drug of formula **B-L"-X** upon cleavage, wherein **X** is an atom or a chemical group, depending on the nature of the cleaving reaction. Preferably, **X** is a hydrogen atom or a hydroxyl group. The free drug released intracellularly may also contain parts (amino acid or peptidic residues) of the antibody molecule, if the linker ist stable, but the antibody molecule is degraded.

In a preferred embodiment, the compound of Formula (I) is less toxic than the toxic compound **B**, **B-X** or **B-L"-X** released upon intracellular cleavage. Methods of testing cytotoxicity in vitro are known in the art (Goldmacher et al., 1985; Goldmacher et al., 1986; see also US 5,208,020, Example 2). Preferably, the compound (I) is 10 times or more, more preferably 100 times or more, or even 1000 times or more less toxic than the free drug released upon cleavage.

Preferably, antibody molecule/maytansinoid conjugates are those that are joined via a disulfide bond, as discussed above, that are capable of delivering maytansinoid molecules. Such cell binding conjugates are prepared by known methods such as modifying monoclonal antibodies with succinimidyl pyridyl-dithiopropionate (SPDP) or pentanoate (SPP; N-succinimidyl-4-(2-pyridyldithio)pentanoate, or N-succinimidyl-5-(2-pyridyldithio)pentanoate) (Carlsson et al, 1978). The resulting thiopyridyl group is then displaced by treatment with thiol-containing maytansinoids to produce disulfide linked conjugates. Alternatively, in the case of the aryldithiomaytansinoids, the formation of the antibody conjugate is effected by direct displacement of the aryl-thiol of the maytansinoid by sulfhydryl groups previously introduced into antibody molecules. Conjugates containing 1 to 10 maytansinoid drugs linked via a disulfide bridge are readily prepared by either method. In this context, it is understood that the decimal number **n** in the formula **A(LB)**_{**n**} is an average number as not all conjugate molecules of a given preparation may have the identical integer of **LB** residues attached to the antibody molecule.

More specifically, a solution of the dithiopyridyl modified antibody at a concentration of 1 mg/ml in 0.1 M potassium phosphate buffer, at pH 7.0 containing 1 mM EDTA is treated with the thiol-containing maytansinoid (1.25 molar equivalent/dithiopyridyl group). The release of pyridine-2-thione from the modified antibody is monitored spectrophotometrically at 343nm and is complete in about 30 min. The antibody-maytansinoid conjugate is purified and freed of unreacted drug and other low molecular weight material by gel filtration through a column of Sephadex G-25. The number of maytansinoids bound per antibody molecule can be determined by measuring the ratio of the absorbance at 252 nm and 280 nm. An average of 1-10 maytansinoid molecules/antibody molecule can be linked via disulfide bonds by this method.

In a preferred aspect, the present invention relates to a conjugate of a CD44v6 specific antibody molecule and a maytansinoid. Herein, "CD44v6 specific" shall mean that the antibody has specific binding affinity to an epitope which is present in a peptide having the amino acid sequence encoded by variant exon v6 of CD44, preferably human CD44. A preferred antibody molecule of the invention specifically binds to peptides or polypetides having or containing the amino acid sequence SEQ ID NO: 1 of the accompanying sequence listing, or an allelic variant of said sequence. Preferably, said antibody molecule has binding specificity for an epitope within said sequence. More preferably, the antibody molecule specifically binds to a peptide having the amino acid sequence SEQ ID NO: 2, even more preferably having the amino acid sequence SEQ ID NO: 3.

Preferably, the antibody molecule in said conjugate is the monoclonal antibody VFF-18 (DSM ACC2174) or a recombinant antibody having the complementary determining regions (CDRs) of VFF-18. More preferably, the said antibody comprises light chains having the amino acid sequence SEQ ID NO: 4, or, alternatively, SEQ ID NO: 8, and heavy chains having the amino acid sequence SEQ ID NO: 6.

The maytansinoid is preferably linked to the antibody by a disulfide moiety and has the formula 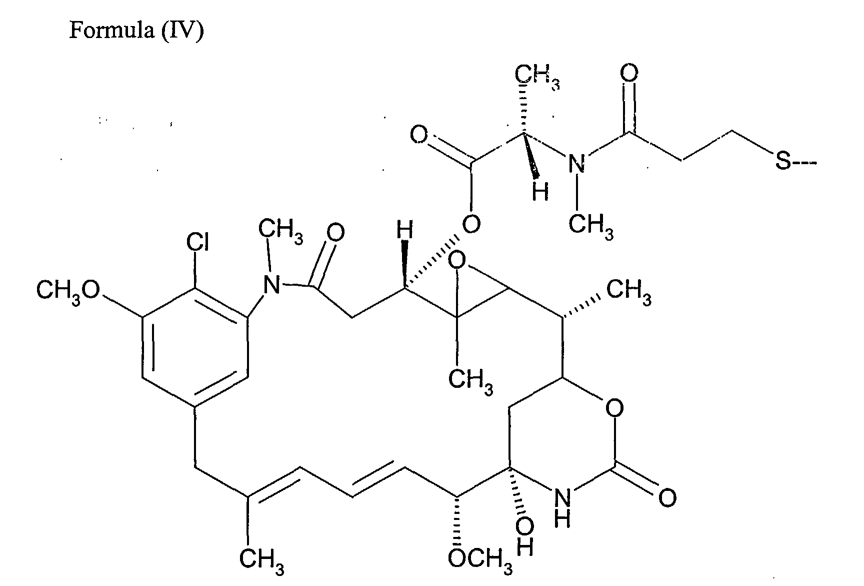 wherein the link to the antibody is through the sulfur atom shown in formula IV to a second sulfur atom present in the antibody molecule. To create such a sulfur atom available for bonding, an antibody molecule may be modified by introduction of a suitable linker as outlined above. Preferably, the maytansinoid is linked to the antibody molecule through a -S-CH₂CH₂-CO-, a ―S-CH₂CH₂CH₂CH₂-CO-, or a -S-CH(CH₃)CH₂CH₂-CO- group. The sulfur atom in such a linker group forms the disulfide bond with the maytansinoid, while the carbonyl function may be bonded to an amino function present on the side chain of an amino acid residue of the antibody molecule.

That way, one or more maytansinoid residues may be linked to an antibody molecule. Preferably, 3 to 4 maytansinoid residues are linked to an antibody molecule.

Most preferred is a conjugate of a CD44v6 specific antibody molecule and a maytansinoid, wherein the antibody comprises light chains having the amino acid sequence SEQ ID NO: 4, and heavy chains having the amino acid sequence SEQ ID NO: 6, and wherein the maytansinoid has the formula 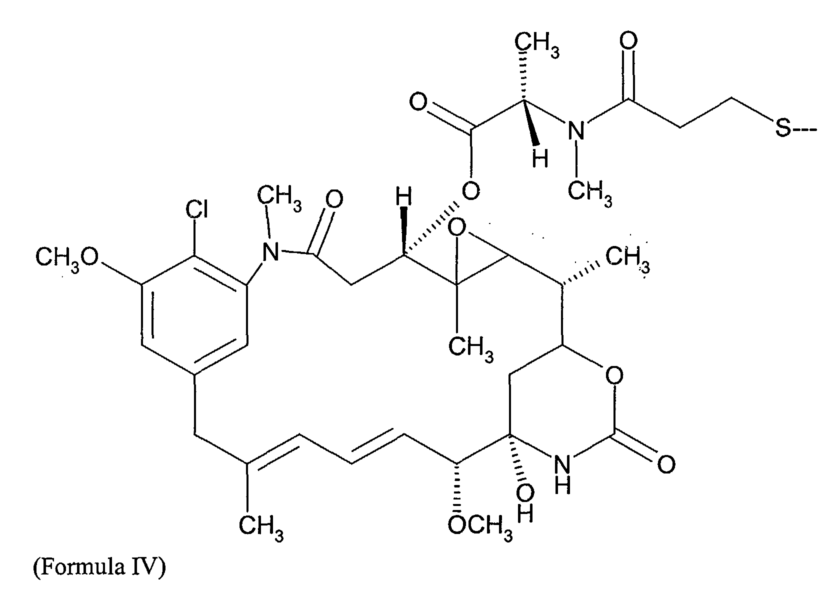 and is linked to the antibody through a disulfide bond. Preferably, the linking group is -S-CH₂CH₂CH₂CH₂-CO- or -S-CH(CH₃)CH₂CH₂-CO-, and the number of maytansinoid residues bound per antibody molecule is 3 to 4.

The conjugate or compound of Formula (I), as defined herein, is preferably formulated into a pharmaceutical composition comprising such conjugate or compound, preferably together with a pharmaceutically acceptable carrier, excipient, or diluent.

Suitable pharmaceutically acceptable carriers, diluents, and excipients are well known and can be determined by those of skill in the art as the clinical situation warrants. In general, the conjugate may be formulated in form of a buffered aqueous solution, using a physiologically acceptable buffer like phosphate buffered saline (PBS; 8 g/l NaCl, 0.2 g/l KC1,1.44 g/l Na₂HPO₄, 0.24 g/l KH₂PO₄ in distilled water, adjusted to pH 7.4 with aqueous HCl), which may contain additional components for solubilisation, stabilisation, and/or conservation, e.g. serum albumin, ethylenediaminetetraacetate (EDTA), benzyl alcohol, or detergents like polyoxyethylenesorbitan monolaurate (Tween 20^{TM}). Examples of suitable carriers, diluents and/or excipients include: (1) Dulbecco's phosphate buffered saline, pH about 7.4, containing about 1 mg/ml to 25 mg/ml human serum albumin, (2) 0.9% saline (0.9% w/v NaCl), and (3) 5% (w/v) dextrose. The formulation may also be in form of a freeze-dried powder which may be reconstituted with water or buffer before administration. Such lyophilisates may contain an bulking agent like, for example, mannitol.

For clinical treatment of cancer, the compound of Formula (I) according to the invention, in particular the conjugate of a CD44v6 specific antibody molecule and a maytansinoid, will be supplied as solutions that are tested for sterility and for endotoxin levels. Examples of suitable protocols of conjugate administration are as follows. Conjugates may be given weekly for 1 to 6 weeks either as an i.v. bolus , or as a continuous infusion for 5 days. Bolus doses can be given in 50 to 100 ml of isotonic saline to which 5 to 10 ml of human serum albumin has been added. Continuous infusions can be given in 250 to 500 ml of isotonic saline, to which 25 to 50 ml of human serum albumin has been added, per 24 hour period. Dosages will be 10 mg to 400 mg/m² of body surface area per application. The dose applied to the patient per administration has to be high enough to be effective, but must be below the dose limiting toxicity (DLT). In general, a sufficiently well tolerated dose below DLT will be considered maximum tolerated dose (MTD). The expert knows how to determine the MTD (Lambert et al., 1998). For weekly administrations, the MTD can be expected to be in the range of 50 to 200 mg/m². Alternatively, intervals between applications may be longer, e.g. two to four weeks, preferably three weeks. In this case, the MTD can be expected to be in the range of 100 to 300 mg/m². Alternatively, application may be in 5 daily doses, followed by a break of several weeks after which treatment may be repeated. In this case, the MTD per administration can be expected to be lower than 100 mg/m². For example, conjugates can be administered as a single i.v. infusion with a rate of 3 mg/min every 21 days.

"Radiotherapy", or radiation therapy, shall mean the treatment of cancer and other diseases with ionizing radiation. Ionizing radiation deposits energy that injures or destroys cells in the area being treated (the target tissue) by damaging their genetic material, making it impossible for these cells to continue to grow. Radiotherapy may be used to treat localized solid tumors, such as cancers of the skin, tongue, larynx, brain, breast, lung or uterine cervix. It can also be used to treat leukemia and lymphoma, i.e. cancers of the blood-forming cells and lymphatic system, respectively.

One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons are machines that produce x-rays of increasingly greater energy. The use of machines to focus radiation (such as x-rays) on a cancer site is called external beam radiotherapy. Gamma rays are another form of photons used in radiotherapy. Gamma rays are produced spontaneously as certain elements (such as radium, uranium, and cobalt 60) release radiation as they decompose, or decay. Another technique for delivering radiation to cancer cells is to place radioactive implants directly in a tumor or body cavity. This is called internal radiotherapy. (Brachytherapy, interstitial irradiation, and intracavitary irradiation are types of internal radiotherapy.) In this treatment, the radiation dose is concentrated in a small area, and the patient stays in the hospital for a few days. Internal radiotherapy is frequently used for cancers of the tongue, uterus, and cervix. A further technique is intraoperative irradiation, in which a large dose of external radiation is directed at the tumor and surrounding tissue during surgery. Another approach is particle beam radiation therapy. This type of therapy differs from photon radiotherapy in that it involves the use of fast-moving subatomic particles to treat localized cancers. Some particles (neutrons, pions, and heavy ions) deposit more energy along the path they take through tissue than do x-rays or gamma rays, thus causing more damage to the cells they hit. This type of radiation is often referred to as high linear energy transfer (high LET) radiation. Radiosensitizers make the tumor cells more likely to be damaged, and radioprotectors protect normal tissues from the effects of radiation. Hyperthermia, the use of heat, may also be used for sensitizing tissue to radiation. Another option involves the use of radiolabeled antibodies to deliver doses of radiation directly to the cancer site (radioimmunotherapy), see below.

Detailed protocols for radiotherapy are readily available to the expert (Cancer Radiotherapy : Methods and Protocols (Methods in Molecular Medicine). Huddart RA (Ed.). Human Press 2002). In one embodiment of the present invention, radiation therapy is provided as external beam radiotherapy. In a preferred embodiment, the external beam radiotherapy is applied as fractionated radiotherapy which means that a defined total radiation dose is applied in small fractions over a certain period of time. For example, 50 Gy of radiation may be applied in 2 Gy fractions over a five-week period, or in 4 Gy fractions. The expert knows how to determine an appropriate dosing and application schedule, depending on the nature of the disease and the constitution of the patient. In particular, the expert knows how to assess dose-limiting toxicity (DLT) and how to determine the maximum tolerated dose (MTD) accordingly.

As an illustrative example, patients with stage I or II CD44v6-positive breast cancer may receive 50 Gy of radiation to the whole breast in 2-Gy fractions over a five-week period after lumpectomy and axillary dissection, in combination with a treatment with a cytotoxic immunoconjugate according to the invention. The radiotherapy protocol may be applied to patients who underwent surgical excision of the primary tumor, with a 1-cm margin of macroscopically normal tissue (lumpectomy), and an axillary dissection. Postoperative mammography can be applied if suspicious microcalcifications are seen before lumpectomy. The surfaces of specimens can be marked with India ink. Patients with a microscopically incomplete excision may receive an additional dose of radiation of either 10 Gy or 26 Gy. Radiotherapy may begin within nine weeks after lumpectomy, or up to six months thereafter. Irradiation of the whole breast may be performed with the use of two tangential megavoltage photon beams (high-energy x-ray or tele-cobalt). A total dose of 50 Gy over a five-week period, with a dose of 2 Gy per fraction, can be delivered at the intersection of the central axes of the beams. The additional dose of 10 or 26 Gy can be given to the center of the area from which the tumor has been excised; in eight equal external-beam fractions with fast electrons or tangential photon fields, or alternatively, by means of an iridium-192 implant with a dose rate of 10 Gy per 24 hours.

As another illustrative example, in patients with advanced, unresectable CD44v6-positive squamous-cell carcinoma of the head and neck (squamous-cell carcinoma of the oral cavity, pharynx, or larynx; stage III or IV), radiotherapy may be combined with treatment with the cytotoxic immunoconjugates as disclosed above. They may receive several cyles of intravenous infusion of he immunoconjugate (e.g. as daily applications for five consecutive days), alternating with radiotherapy e.g. in three two-week courses (20 Gy per course; 2 Gy per day, five days per week).

As another illustrative example, thoracic radiotherapy may be combined with cytotoxic immunotherapy according to the present invention in patients with completely resected stage II or IIIa CD44v6-positive non-small-cell lung cancer. After surgical staging and resection of the tumor (usually by lobectomy or pneumonectomy) cytotoxic immunoconjugate as described above may be administered concurrently with radiotherapy (e.g. a total of 50.4 Gy, given in 28 daily fractions of 1.8 Gy). The initial portion of the radiation treatment can be administered with anteroposterior and posteroanterior portals with a limit of 36 to 42 Gy. The remainder of the treatment may involve the same target volume but use a lateral or oblique field arrangement that prevents any level of the spinal cord from receiving more than 45 Gy. An additional 10.8 Gy (six fractions of 1.8 Gy) can be administered to nodal levels at which extracapsular extension of nodal metastases may be documented. Treatment with the immunoconjugate as outlined above may be initiated within 24 hours after radiotherapy has begun.

In a further example, patients with CD44v6-positive bulky stage IB cervical cancers may receive radiotherapy in combination with cytotoxic immunoconjugate as outlined above, followed in by adjuvant hysterectomy. The cumulative dose of external pelvic and intracavitary radiation may be 75 Gy to point A (cervical parametrium) and 55 Gy to point B (pelvic wall). Immunoconjugate may be given during external radiotherapy as weekly intravenous infusions, and adjuvant hysterectomy may be performed three to six weeks later. Patients may undergo external irradiation, intracavitary brachytherapy, and extrafascial hysterectomy. Pelvic radiation may be delivered with the four-field technique with x-ray accelerators of at least 4-MV photons at a distance of at least 100 cm. The treatment field may be set to extend 3 cm beyond the known extent of disease and to encompass iliac and lower common iliac lymph nodes. Fractions of 1.8 to 2.0 Gy may be delivered 5 days a week over a period of 4 1/2 to 5 weeks, for a total dose of 45 Gy. External irradiation may be withheld if the white-cell count falls below 1000 per cubic millimeter and may be resumed once the count rose above that level. Low-dose brachytherapy may be performed in one or two intracavitary applications after the completion of pelvic radiotherapy. Standard Fletcher-Suit or Henschke applicators may be used. The dose to point A (a reference location 2 cm lateral and 2 cm superior to the cervical os) may be 30 Gy, for a cumulative dose of 75 Gy, and the cumulative dose to point B (the pelvic wall) may be 55 Gy.

In another embodiment, the cytotoxic immunoconjugate may be combined with a radioimmunoconjugate, i.e. an antibody molecule which is linked to one or more radioisotopes. Preferably, the radioimmunoconjugate is specific for the same antigen as the cytotoxic immunoconjugate because this provides an optimal concentration of the both agents at the site of the tumor. Therefore, in a particular preferred embodiment, both conjugates are specific for CD44v6 as outlined above. In a preferred example, both conjugates are based on the antibody BIWA 4 disclosed above. Among the radioisotopes, gamma, beta and alpha-emitting radioisotopes may be used as the active agent of radiation therapy. β-emitting radioisotopes are preferred as therapeutic radioisotopes. ¹⁸⁶Rhenium, ¹⁸⁸Rhenium, ¹³¹Iodine and ⁹⁰Yttrium have been proven to be particularly useful β-emitting isotopes to achieve localized irradiation and destruction of malignant tumor cells. Therefore, radioisotopes selected from the group consisting of ¹¹⁶ Rhenium, ¹⁸⁸Rhenium, ¹³¹Iodine and ⁹⁰Yttrium are particularly preferred as radiation sources.

There are numerous methods available in the art to link a radioisotope to an antibody. For example, for the radioiodination of the antibody, a method as disclosed in WO 93/05804 may be employed. Another option is to to use a linker moelcule between te antibody and the radioisotope, e.g. MAG-3 (US 5,082,930, EP 0 247 866), MAG-2 GABA (US 5,681,927, EP 0 284 071), and N2S2 (phenthioate, US 4,897,255, US 5,242,679, EP 0 188 256). Preferably, the radioimmunoconjugate has a specific activity of from about 0.5 to about 15 mCi/mg, or from about 0.5 to about 14 mCi/mg, preferably about 1 to about 10 mCi/mg, preferably about 1 to about 5 mCi/mg, and most preferably 2 to 6 mCi/mg or 1 to 3 mCi/mg. The radio immunoconjugate may be applied via an intravenous or other route, e.g. systemically, locally or topically to the tissue or organ of interest, depending on the location, type and origin of the tumor. Depending on the desired duration and effectiveness of the treatment, pharmaceutical antibody compositions may be administered once or several times, also intermittently, for instance on a daily basis for several days, weeks or months and in different dosages. The amount of the radioimmunoconjugate applied depends on the nature of the disease. The dose of radioactivity applied to the patient per administration has to be high enough to be effective, but must be below the dose limiting toxicity (DLT). For pharmaceutical compositions comprising radiolabeled antibodies, e.g. with ¹⁸⁶Rhenium, the maximally tolerated dose (MTD) has to be determined which must not be exceeded in therapeutic settings. Application of radiolabeled antibody to cancer patients may then be carried out by repeated (monthly or weekly) intravenous infusion of a dose which is below the MTD (See e.g. Welt et al. (1994) *J. Clin. Oncol.* **12**: 1193-1203). Multiple administrations are preferred, generally at weekly intervals; however, radiolabelled materials should be administered at longer intervals, i.e., 4-24 weeks apart, preferable 12-20 weeks apart. The artisan may choose, however, to divide the administration into two or more applications, which may be applied shortly after each other, or at some other predetermined interval ranging, e.g. from 1 day to 1 week. In general, the radioactivity dose per administration will be between 30 and 75 mCi/m² body surface area (BSA). Thus, the amount of radiolabelled antibody in the pharmaceutical composition according to the invention, preferably labelled with ¹⁸⁶Rhenium, ¹⁸⁸Rhenium, ^{99m}Technetium, ¹³¹Iodine, or ⁹⁰Yttrium, most preferably labelled with ¹⁸⁶Rhenium, to be applied to a patient is 10, 20, 30, 40, 50 or 60 mCi/m², preferably 50 mCi/m².

In the context of this invention, "in combination with" shall mean that the compound of Formula (I), in particular the conjugate of a CD44v6 specific antibody molecule and a maytansinoid, and the radiotherapy are applied to the patient in a regimen wherein the patient may profit from the beneficial effect of such a combination. In particular, both treatments are applied to the patient in temporal proximity. In a preferred embodiment, both treatments are applied to the patient within four weeks (28 days). More preferably, both treatments are applied within two weeks (14 days), more preferred within one week (7 days). In a preferred embodiment, the two treatments are applied within two or three days. In another preferred embodiment, the two treatments are applied at the same day, i.e. within 24 hours. In another embodiment, the two treatments are applied within four hours, or two hours, or within one hour. In another embodiment, the two treatments are applied in parallel, i.e. at the same time, or the two administrations are overlapping in time. For example, the cytotoxic conjugate and the radioimmunoconjugate may be infused at the same time, or the infusions may be overlapping in time. Or, the cytotoxic conjugate may be infused at the same time a the radiation is applied. If two drugs are administered at the same time, e.g. in the case of combining the cytotoxic with a radioimmunoconjugate, they may be formulated together in one single pharmaceutical preparation, or they may be mixed together immediately before administration from two different pharmaceutical preparations, for example by dissolving or diluting into one single infusion solution. In another embodiment, the two drugs are administered separately, i.e. as two independent pharmaceutical compositions. In one preferred embodiment, administration of the two treatments is in a way that tumour cells within the body of the patient are exposed to effective amounts of the cytotoxic drug and the radiation at the same time. In another preferred embodiment, effective amounts of both the cytotoxic drug and the radiation are present at the site of the tumour at the same time. The present invention also embraces the use of further agents, which are administered in addition to the combination as defined. This could be, for example, one or more further chemotherapeutic agent(s). It could also be one or more agent(s) applied to prevent, suppress, or ameliorate unwanted side effects of any of the other drugs given. For example, a cytokine stimulating proliferation of leukocytes may be applied to ameliorate the effects of leukopenia or neutropenia.

Dose, route of administration, application scheme, repetition and duration of treatment will in general depend on the nature of the disease (type, grade, and stage of the tumor etc.) and the patient (constitution, age, gender etc.), and will be determined by the medical expert responsible for the treatment. With respect to the possible doses for the components of the disclosed combination which are described above, it is clear that the medical expert responsible for the treatment will carefully monitor whether any dose-limiting toxicity or other severe side effects occur and undertake the necessary steps to manage those.

The present invention is of particular advantage for the treatment of squameous cell carcinomas expressing CD44 antigen, preferably CD44v6. It is in particular suitable for head and neck squameous cell carcinoma, esophagus squameous cell carcinoma, lung squameous cell carcinoma, skin squameous cell carcinoma, or cervix squameous cell carcinoma. Furthermore, it is of particular advantage for the treatment of adenocarcinomas expressing CD44 antigen, perferably CD44v6. It is in particular suitable for breast adenocarcinoma, lung adenocarcinoma, pancreas adenocarcinoma, colon adenocarcinoma, or stomach adenocarcinoma. Besides treatment of clinically apparent malignant disease, therapeutic application according to the invention may be particularly advantageous as an adjuvant to surgical intervention, to treat minimal residual disease.

In a further aspect, the present invention relates to a pharmaceutical composition comprising a compound **A(LB)**_{**n**} or conjugate as defined above together with a radioimmunotherapeutic agent as defined above, and optionally further comprising one or more pharmaceutically acceptable carrier(s), diluent(s), or excipient(s).

In a further aspect, the present invention relates to a kit comprising, in separate pharmaceutical compositions, a compound **A(LB)**_{**n**} or conjugate as defined above, and a radioimmunotherapeutic agent as defined above.

In a further aspect, the present invention relates to the use of a radioimmunotherapeutic agent for the preparation of a pharmaceutical composition for the treatment of cancer, wherein said radioimmunotherapeutic agent is used or is for use in combination with a compound of Formula **A(LB)**_{**n**} or conjugate as defined above.

### References

Aguiar DJ, Knudson W, and Knudson CB. Internalization of the hyaluronan receptor CD44 by chondrocytes. Exp.Cell.Res. 252: 292-302, 1999.
Aujame L, Geoffroy F, Sodoyer R. High affinity human antibodies by phage display. Hum. Antibodies 1997;8(4):155-68, 1997.
Bazil V and Horejsi V. Shedding of the CD44 adhesion molecule from leukocytes induced by anti-CD44 monoclonal antibody simulating the effect of a natural receptor ligand. J. Immunol. 149 (3):747-753, 1992.
Blakey and Thorpe, Antibody, Immunoconjugates and Radiopharmaceuticals, 1: 1-16, 1988.
Blattler et al, Biochem. 24: 1517-1524, 1985.
Breitling F, Duebel S: Recombinant Antibodies. John Wiley, New York, 1999.
Burnett et al., J. Med. Chem. 21: 88, 1978.
Bums, Anal. Prof. Drug Subs. 1: 463-480, 1972.
Burris et al. J. Clin. Oncol. 11: 950, 1993.
Carlsson et al, Biochem. J. 173: 723-737, 1978.
Catty D. Antibodies. Oxford IR Press, 1988.
Chari RVJ, Martell BA, Gross JL, Cook SB, Shah SA, Blättler WA, McKenzie SJ, Goldmacher VS. Immunoconjugates containing novel maytansinoids: promising anticancer drugs. Cancer Research 52: 127-31, 1992.
Chari RVJ, Derr SM, Steeves RM, Widdison WC: Dose-response of the anti-tumor effect of HLTN901-DM1 against human small cell lung cancer xenografts. Proceedings of the American Association of Cancer Research (April 1-5, 2000) 41:(April 1-5) Abs 4405, 2000.
Chen et al., Proc. Annu. Meet. Am. Assoc. Cancer Res. 41: Abs 4578, 2000.
Chothia and Lesk. J. Mol. Biol. 196: 901-917, 1987.
Chou et al., Proc. Natl. Acad. Sci. USA 95(16): 9642-9647, 1998.
Decosterc et al., Anti-Cancer Drugs 10(6): 537-543, 1999.
De-Ines et al., Cancer Res. 54(1): 75-84, 1994.
Denis et al. J. Org. Chem. 56: 6939, 1991.
Frank et al. Methods Enzymol. 154: 221-249, 1984.
Gait,M.J., Oligonucleotide Synthesis. A Practical Approach. IRL Press, Oxford, UK, 1984.
Goetz A D et al., Proc. Am. Soc. Clin. Oncol., 20:Pt 1 (Abs 419), 2001.
Goldbrunner et al., J. Med. Chem. 40(22): 3524-3533, 1997.
Goldmacher et al., J. Immunol. 135: 3648-3651, 1985.
Goldmacher et al., J. Cell Biol. 102: 1312-1319, 1986.
Goodsell, The Oncologist 5: 345-346, 2000.
Gorman et al., J. Am. Chem. Soc. 81: 4745-4754, 1959.
Güssow D, Seemann G. Humanization of monoclonal antibodies. Methods Enzymol. 203: 99-121, (1991).
Guo YJ, Liu G, Wang X, Jin D, Wu M, Ma J, and Sy MS. Potential use of soluble CD44 in serum as indicator of tumor burden and metastasis in patients with gastric or colon cancer. Cancer Res 54 (2): 422-426, 1994.
Harlow L D. Antibodies. Cold Spring Harbor Lab.,1988.
Hayden et Mandecki. Gene synthesis by serial cloning of oligonucleotides. DNA 7(8): 571-7, 1988.
Heider, K.-H., Hofmann, M., Horst, E., van den Berg, F., Ponta, H., Herrlich, P., and Pals, S.T. A human homologue of the rat metastasis-associated variant of CD44 is expressed in colorectal carcinomas and adenomatous polyps. J. Cell Biol. 120: 227-233, 1993.
Heider KH, Mulder JWR, Ostermann E,Susani S, Patzelt E, Pals ST, Adolf GRA. Splice variants of the cell surface glycoprotein CD44 associated with metastatic tumor cells are expressed in normal tissues of humans and cynomolgus monkeys. Eur. J. Cancer 31A: 2385-2391, 1995.
Heider KH, Sproll M, Susani S, Patzelt E, Beaumier P, Ostermann O, Ahorn H, Adolf GRA. Characterization of a high affinity monoclonal antibody antibody specific for CD44v6 as candidate for immunotherapy of squamous cell carcinomas. Cancer Immunology Immunotherapy 43: 245-253, 1996.
Hofmann, M., Rudy, W., Zöller, M., Tölg, C., Ponta, H., Herrlich P., and Günthert, U. CD44 splice variants confer metastatic behavior in rats: homologous sequences are expressed in human tumor cell lines. Cancer Res. 51: 5292-5297, 1991.
Holton et al. J. Am. Chem. Soc. 116: 1597, 1599, 1994.
Horsman et al., Proc. Annu. Meet. Am. Assoc. Cancer Res. 39: Abs 1142, 1998.
Hoshi et Castaner, Drugs Future 18(11): 995-996, 1993.
Johnson S, Bird R E. Construction of single-chain derivatives of monoclonal antibodies and their production in *Escherichia coli.* Methods Enzymol. 203: 88-98, 1991.
Kabat E. A., Wu T. T., Perry H. M., Gottesman K. S. and Foeller C. Sequences of Proteins of Immunological Interest (5th Ed.). NIH Publication No. 91-3242. U.S. Department of Health and Human Services, Public Health Service, National Institutes of Health, Bethesda, MD, 1991.
Klotz et al, Arch. Biochem. Biophys. 96: 605, 1962.
Koopman, G., Heider, K.-H., Horts, E., Adolf, G. R., van den Berg, F., Ponta, H., Herrlich, P., Pals, S. T. Activated human lymphocytes and aggressive Non-Hodgkin's lymphomas express a homologue of the rat metastasis-associated variant of CD44. J. Exp. Med. 177: 897-904, 1993.
Kortt AA, Dolezal O, Power BE, Hudson PJ. Dimeric and trimeric antibodies: high avidity scFvs for cancer targeting. Biomol. Eng. 18(3), 95-108, 2001.
Kreitman R J Hansen H J, Jones A L, FitzGerald D J P, Goldenberg D M, Pastan I. *Pseudomonas* exotoxin-based immunotoxins containing the antibody LL2 or LL2-Fab' induce regression of subcutaneous human B-cell lymphoma in mice. Cancer Res. 53: 819-825, 1993.
Krivit et al., Cancer Chemother. Pharmacol. 2: 267, 1979.
Lambert et al, Biochem. 22: 3913-3920, 1983.
Lambert JM, Derr SM, Cook S, Braman G, Widdison W, Chari RVJ. Pharmacokinetics, in vivo stability, and toxicity of the Tumor-activated prodrug, C242-DM1, a novel colorectal cancer agent. Proceedings of the American Association of Cancer Research 39: Abs 3550, 1998.
Lee et al., Clin Cancer Res 7(5): 1429-1437,2001.
Liu et al., Biochem. 18: 690, 1979.
Liu C, Tadayoni BM, Bourret LA, Mattocks KM, Derr SM, Widdison WC, Kedersha NL, Ariniello PD, Goldmacher VS, Lambert JM, Blättler WA, Chari RVJ. Eradication of large colon tumor xenografts by targeted delivery of maytansinoids. Proc. Natl. Acad. Sci. USA 93: 8618-23, 1996.
Lu et Meistrich, Cancer Res. 39: 3575, 1979.
Mangeney et al., Tetrahedron 35: 2175, 1979.
Mangatal et al. Tetrahedron 45: 4177, 1989.
Martin S, Jansen F, Bokelmann J, and Kolb H. Soluble CD44 splice variants in metastasizing human breast cancer. Int. J. Cancer 74 (4): 443-445, 1997.
Marty et al., Nouv. Rev. Fr. Hematol. 31: 77-84, 1989.
Mekhail et al., Proceedings of the American Society for Clinical Oncology, 21:1(Abs 408), 2002.
Mross et al., Onkologie 19(6): 490-495, 1996.
Muhlradt et al., Cancer Res 57(16): 3344-3346, 1997.
Muhtadi et Afifi, Analytical Profiles of Drug Substances and Excipients 21: 611-658, Brittain (Ed.), Academic Press, San Diego, 1992.
Neus et al., J. Am. Chem. Soc. 86: 1440, 1964.
Nicolaou et al., Bioorg. Med. Chem. 7(5): 665-697, 1999.
Ohsumi et al., J. Med. Chem. 41(16): 3022, 1998.
Owellen et Donigian, J. Med. Chem. 15: 894, 1972.
Owellen et al., Cancer Res. 37: 2603, 1977.
Pettit et al., J. Med. Chem. 43(14): 2731-2737, 2000.
Rahmani et al., Cancer Res. 47: 5796-5799, 1987.
Runowicz et al. Cancer 71: 1591-1596, 1993.
Screaton, G.R., Bell, M.V., Jackson, D.G., Cornelis, F.B., Gerth, U., and Bell, J. I. Genomic structure of DNA encoding the lymphocyte homing receptor CD44 reveals at least 12 alternatively spliced exons. Proc. Natl. Acad. Sci. U.S.A. 89: 12160-12164, 1992.
Shin S-U, Morrison S L. Production and properties of chimeric antibody molecules. Methods Enzymol. 178: 459-476, 1989.
Sieber et al., Cancer Treat. Rep. 60: 127, 1976.
Sliutz G, Tempfer C, Winkler S, Kohlberger P, Reinthaller A, and Kainz C. Immunohistochemical and serological evaluation of CD44 splice variants in human ovarian cancer. Br.J.Cancer 72: 1494-1497,, 1995.
Stemmer et al. Single-step assembly of a gene and entire plasmid from large numbers of oligodeoxyribonucleotides. Gene 164(1): 49-53, 1995.
Stroomer JW, Roos JC, Sproll M, Quak JJ, Heider KH, Wilhelm BJ, Castelijns JA, Meyer R, Kwakkelstein MO, Snow GB, Adolf GR, van Dongen GA. Safety and biodistribution of 99mTechnetium-labeled anti-CD44v6 monoclonal antibody BIWA 1 in head and neck cancer patients. Clin. Cancer Res. 6 (8): 3046-55, 2000.
Suffness [Ed.]. Taxol®. Science and Applications. CRC Press, Boca Raton, 1995.
Tölg, C., Hofmann, M., Herrlich, P., and Ponta, H. Splicing choice from ten variant exons establishes CD44 variability. Nucleic Acids. Res. 21: 1225-1229, 1993.
Tolcher AW et al. SB-408075, a maytansinoid immunoconjugate directed to the C242 antigen: a phase I pharmacokinetic and biologic correlative study. Poster presented at 11^{th} Symp. on new drugs in cancer therapy (Nov 7-10, 2000 in Amsterdam), 2000.
Urlaub and Chasin. Proc. Natl. Acad. Sci. U.S.A. 77(7): 4216-20, 1980.
Urlaub et al., Cell 33: 405-412, 1983.
Valluri et al., Org Lett 3 (23): 3607-3609, 2001.
Van-den-Berge et al., Anticancer Res. 13(1): 273-277, 1993.
Verel et al., Int. J. Cancer 99: 396-402, 2002.
Vite et al., ACS Meeting 2002,223rd:Orlando(MEDI 18), 2002.
Wang et al., AACR NCI EORTC Molecular Targets and Cancer Therapeutics 2001, October 29-November 2(Abs #781), 2001.
Wani et al., J. Am. Chem. Soc. 93: 2325, 1971.
Welt et al., J. Clin. Oncol. 12: 1193-1203, 1994
White et al., Org. Lett. 1(9): 1431-1434, 1999.
Winter, G., Griffith, A. D., Hawkins, R. E., Hoogenboom, H. R. Making antibodies by phage display technology. Ann. Rev. Immunol. 12: 433-455, 1994.
Worrell et al, Anti-Cancer Drug Design 1: 179-184, 1986.
Yamaguchi et al., Cancer Res. 62 (2): 466-471, 2002.
Ye et al., Gene synthesis and expression in E. coli for pump, a human matrix metalloproteinase. Biochem. Biophys. Res. Commun. 186(1):143-9, 1992.
Zhu et Panek, Org. Lett. 2 (17): 2575-2578, 2000.

### Examples

### Example 1: In vitro cell proliferation assay

For determination of viable cells the Cell Titer 96® AQᵤₑₒᵤₛ non-radioactive cell proliferation assay (Promega) was used. 5000 cells per well were seeded into 96-well plates in 90 µl medium without phenole red. Cells were allowed to settle down for 1 to 3 h and then serial dilutions of the immunoconjugate in 10 µl PBS were added. Cells without immunoconjugate served as negative control. Cells were incubated for 4 days at 37°C in a humified 5% CO₂ atmosphere and then 20 µl MTS/ PMS were added according to the manufacturer's recommendation. After additional 1 to 4 h incubation at 37°C the absorbance at 490 nm was recorded using an ELISA plate reader. For each cell line triplicates were analyzed. The percentage of the surviving cell fraction and the IC50 value were calculated using the GraphPad Prism® (Version 3.0) software..

### Example 2: Manufacturing of BIWI 1

Humanised recombinant antibodies BIWA 4 and BIWA 8 which have binding specificity for an epitope within SEQ ID NO: 1 were linked to the maytansinoid DM1 as described below. The conjugate of BIWA 4 with DM1 was designated BIWI 1.

**Generation of stably transfected cell lines.** The genes coding for the light and heavy chains of BIWA 4, SEQ ID NO: 5 and SEQ ID NO: 7, were ligated into expression vector pAD-CMV1 (WO92/01055; NCBI GenBank Accession No. A32111) or pAD-CMV19 (NCBI GenBank Accession No. A32110). In the second antibody BIWA 8, the light chain was coded by a gene having SEQ ID NO: 9, while the heavy chain was the same as in BIWA 4. Stably transfected cell lines were generated by electroporation as follows. CHO DUX/57ss (dhfr negative mutant of Chinese Hamster Ovary cells, adapted for serum free suspension culture) were used. After trypsinisation and inactivation of trypsin with RPMI-10 (90% RPMI 1640, 10% heat inactivated fetal calf serum), cells were washed once with RPMI-0 (RPMI 1640 without serum), and 1x10⁷ cells were resuspended in 0.8 ml RPMI-0.

After addition of the linearised DNA (20 µg per plasmid; cotransfection of vectors coding for light and heavy chain) the cells were electroporated using a Hoefer Electroporator under the following conditions: 1080 µF, 320 V, 1000 msec, 1 pulse. Cells were allowed to stand for 5 min, and were then diluted to 12500 cells/ml and 2500 cells/ml in alfa-MEM 10d (90% MEM alfa without ribonucleosides and without desoxyribonucleosides (GIBCO BRL), 10% heat inactivated dialysed fetal calf serum). The cells were seeded into 96 well microtiter plates (200 µl/well, corresponding to 2500 and 500 cells/well respectively). Clones appeared after 10 days. Only the plates with 500 cells/well were followed up (3-6 clones/well). After 14-15 days, supernatants from each well were tested in a κ/γ ELISA. 53 clones were seeded in 12 well plates in alfa-MEM 10d. After 3-6 days (depending on the confluency of the cells) supernatants were tested again in the κ/γ ELISA (serial dilutions) and quantitated using a human IgGl standard. Cells were frozen and stored in liquid nitrogen. IgG contents of the 53 clones ranged from 12 - 417 ng/ml. 10 clones with the highest expression level were selected and subcloned as follows: Cells of each clone were seeded into 96 well microtiter plates with densities of 1 and 5 cells/well in 100µl/well alfa-MEM 10d (1 plate for each clone and each density). 8 days later supernatants were diluted 1:2 and 100 µl of this dilution tested in the κ/γ ELISA and quantitated using a BIWA 4 preparation as standard. 5 subclones of each clone were transferred to 12 well plates. The IgG content ranged from 1.3 - 908 ng/ml. 14 clones with the highest expression level (384 - 908 ng/ml) were used for amplification with methotrexate as follows: Clones were initially cultured in 25 cm² flasks containing alfa-MEM 10d with 20, 50 and 100 nM methotrexate. After the outgrowth of clones the supernatants were tested in the κ/γ ELISA. In subsequent rounds of amplification the methotrexate concentration was raised up to 2000 nM. Initially the highest expression level ranged from 10.5-14.8 µg/ml (clone A31/100, 100 nM methotrexate). Further amplification with a methotrexate concentration of 500 nM gave an expression of 19-20 µg/ml (A31/500).

**Purification of antibody.** Antibody was purified from cell culture supernatant as follows. Antibody containing tissue culture supernatant was applied onto a 5 ml protein A sepharose column with a flow rate of 80-90 ml/h at 4 °C. After washing with 50 ml binding buffer (0.1 M sodium phosphate pH 7.5), the Ig fraction was eluted with elution buffer (0.1 M glycine-HCl pH 2.7). Absorption at 280 nm was monitored.

**Modification of BIWA 4 with SPP to form BIWA 4-SS-Py.** BIWA 4 was supplied in liquid form at a concentration of 5 mg/mL in a PBS formulation containing Tween 20. Prior to coupling of DM1 to the MAb the Tween 20 was removed. The MAb solution (40 mL) was diluted 15-fold with 25 mM MES buffer, pH 5.6, containing 50 mM NaCl (MES buffer) and then loaded onto a column (12.5 mL) of Sepharose S equilibrated in MES buffer (flow rate: 100 cm/hr). The column was washed with 10 column volumes of MES buffer. The antibody was eluted with MES buffer containing 400 mM NaCl. The antibody solution was dialysed against 50 mM potassium phosphate buffer, pH 6.5 containing 50 mM NaCl and 2 mM EDTA (Buffer A).The BIWA 4 antibody was modified using SPP ((2-Pyridyl)-5-dithiopentanoic acid N-hydroxy succinimid ester) to introduce dithiopyridyl groups. The MAb in Buffer A (185 mg, 8 mg/mL) was modified with a 7-fold molar excess of SPP in EtOH (5% v/v of MAb solution). The reaction proceeded for 90 minutes at ambient temperature. The reaction mixture was then subjected to gel filtration chromatography through Sephadex G25F (2.6 x 31.5 cm column, 167 mL) equilibrated in Buffer A. MAb-containing fractions were pooled and the degree of modification was determined by measuring the absorbance at 280 nm and the change in absorbance at 343 nm caused by the release of 2-mercaptopyridine by the addition of DTT. The concentration of released 2-mercaptopyridine was calculated using an ε_{343 nm} of 8080 M⁻¹cm⁻¹, and the concentration of MAb was calculated using an ε_{280 nm} of 224,000 M⁻¹ cm⁻¹ after the absorbance at 280 nm has been corrected for the contribution from 2-mercaptopyridine. (2-mercaptopyridine A_{280 nm} = A_{343 nm} x 5100/8080). Recovery of the MAb was 99.6% with 5.5 releasable 2-mercaptopyridine groups linked per MAb molecule.

**Conjugation of BIWA 4-SS-Py with DM1.** The above modified MAb (184 mg) in Buffer A was conjugated at 2.5 mg MAb/mL using a 1.7-fold molar excess of DM1 over releasable 2-mercaptopyridine groups. DM1 was added in DMA (3% v/v of MAb solution) and the reaction mixture was incubated at ambient temperature for 29 hours. The conjugate was then isolated by gel filtration chromatography on a column of Sephacryl S300 HR equilibrated in PBS (5 x 50 cm column, 980 mL, flow rate of 10 cm/hr). The conjugate eluted as a single peak at the position of monomeric MAb with a small amount of protein eluting earlier. Fractions were assayed for the number of DM1 molecules linked per MAb molecule. (Linked DM1 molecules were determined by measuring the absorbance at both 252 nm and 280 nm). Based on the results, fractions representing 63-77% of the column volume were pooled. The DM1/MAb ratio in the pooled solution was found to be 3.1 and the yield of conjugated BIWA 4 was 75% based on starting MAb. The conjugate, BIWI 1, was evaluated by SDS-PAGE performed under non-reducing conditions and found to be composed primarily of a monomer species (>95%) with a minor amount (<5%) of dimeric conjugate.

### Example 3: Analysis of in vitro binding of BIWI 1.

The binding of BIWA 4 antibody and BIWI 1 conjugate to antigen-positive FaDu cells was determined. Cells (1-2 x 10⁻⁵) were incubated in 96-well plates with varying concentrations of antibody or conjugate on ice for 1 hour. The test article was washed from the plate and FITC-labeled anti-human IgG was added and the incubation on ice was continued in the dark for 1 hour. After washing, the cells were fixed with 1% paraformaldehyde and analyzed on a fluorescence activated cell sorter (FACS). BIWA 4 antibody binds with an apparent K_{D} of 1 x 10⁻⁹ M and BIWI 1 binds with an apparent K_{D} of 1.8 x 10⁻⁹ M. Thus, conjugation with DM1 alters the binding affinity of the antibody only slightly if at all.

### Example 4: Combined application of BIWI 1 and radiotherapy to nude mice carrying human tumor tisse

BIWI 1, BIWA 4 and phosphate buffered saline (PBS) were applied intravenously (i.v.) in one of the tail veins. BIWI 1 was applied in doses of 50 µg and 100 µg DM1/ kg body weight (b.w.)/ d. Control animals were treated either with BIWA 4 (antibody control, 7 mg/kg b. w./ d) or PBS.

**Animals:** The experiments were performed using 7-14 week old male and female NMRI (nu/nu) mice from the specific pathogen-free animal breeding facility of the Experimental Center of the Medical Faculty of the University of Dresden. The animal facilities and the experiments were approved according to the German animal welfare regulations. The microbiological status of the animals was regularly checked by the veterinarians of the facility. The animal rooms provided day light plus a 12h light- 12h dark electric cycle (light-on time 07.00 a.m.) and a constant temperature of 26°C and relative humidity of 50-60%. The animals were fed a commercial laboratory animal diet and water *ad libitum.* To immunosuppress the nude mice further, they were whole-body irradiated 2 days before tumor transplantation with 4 Gy using 200 kV X-rays (0.5 mm Cu) at a dose rate of about 1 Gy/min.

**Tumor:** FaDu is an established human hypopharyngeal squamous cell carcinoma line, kept in high passage by the American Type Culture Collection (Rockville, MD). In nude mice FaDu grows as an undifferentiated carcinoma with a volume doubling time (VDT) between 100 mm³ and 400 mm³ of about 4 days. In extensive series of quantitative tumor transplantation and of radiation tumour control assays FaDu tumours have been shown to evoke no or only a very low level of residual immune reactivity in nude mice. For the experiments small tumor pieces were transplanted s.c. into the right hindleg of anaesthetised mice.

**Combination of BIWI 1 with fractionated irradiation:** Local irradiations were given under ambient conditions without anaesthesia to air breathing animals (200 kV X-rays, 0.5 mm Cu, at a dose rate of 1.1 Gy/min). Up to five animals were irradiated simultaneously in specially designed jigs. For the treatments the mice were immobilised in a plastic tube fixed on a lucite plate. The tumor-bearing leg was held positioned in the irradiation field by a foot-holder distal to the tumor. Tumor volume at the start of irradiation were around 100 mm³. Tumors were irradiated with 5 fractions of 4 Gy in 5 days. The animals were randomized over the experimental matrix in groups of 5, aiming for about 15 animals (range 14 to 15 animals) for each treatment group. Irradiation protocols were started every second day. Animals received either BIWI 1 (50 µg DM1/ kg/ d), BIWI 1 (100 µg DM1/kg/ d), BIWA 4 (7 mg/ kg/ d) or PBS two hours after each irradiation for 5 consecutive days,. The report includes data obtained from a total of 59 irradiated tumors. In addition 57 unirradiated FaDu tumors were treated with BIWI 1 (50 µg/ kg/ d), BIWI 1 (100 µg/ kg/ d), BIWA 4 (7 mg/ kg/ d) or PBS for 5 consecutive days.

Determinations of tumor volume and tumor growth delay: Animals were observed until the mean diameter of the untreated or recurrent tumors exceeded 12-15 mm, until death or until day 120 after end of treatment. Animals that appeared to suffer were killed before reaching these endpoints. Tumor diameters were measured every second day. Tumor volumes were determined by the formula of a rotational ellipsoid π/6 x a x b², where a is the longest and b is the perpendicular shorter tumor axis. Conversion of tumor volumes to tumor mass (mg) was performed by a calibration curve based on excision weights. Tumor growth delay was determined from individual growth curves as the time that a regrowing tumor needed to reach 5 and 10 times the start volume (t_{V5}, t_{V10}).

**Statistics:** Medians and 95% confidence intervals (CI) of the tumor growth delay were calculated according to Sachs (Angewandte Statistik, 7. Aufl., Springer 1992). Experimental groups were compared using the Mann-Whitney-U test and a commercial software package (SPSS for Windows 9.0, SPSS Inc., 1999).

### Results:

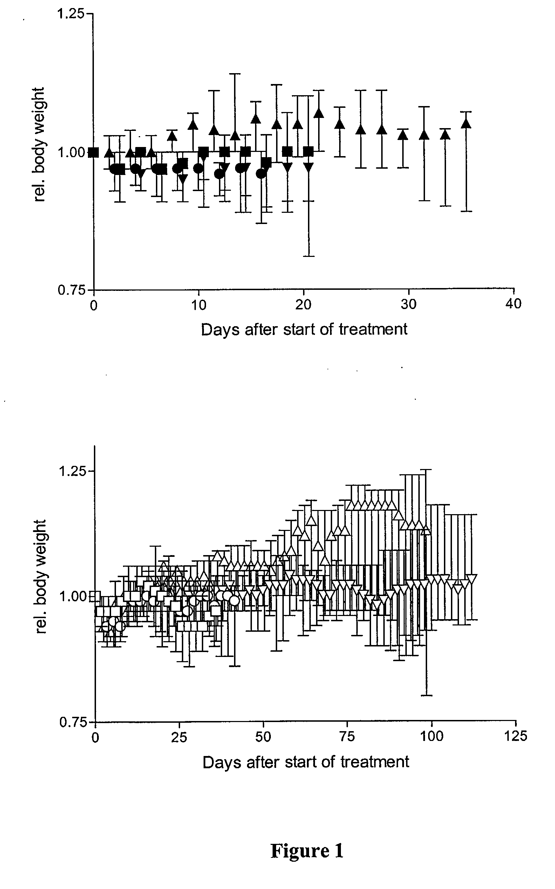

Tolerability: BIWI 1 was well tolerated by the animals. No toxicity was observed after application of BIWI 1 or BIWA 4. Body weight of irradiated and unirradiated animals during the treatment showed some minor and temporary changes without significant differences between the BIWI 1, BIWA 4 and PBS treated group (Figure 1). Acute skin reactions after combined treatment were not enhanced compared with irradiation alone.

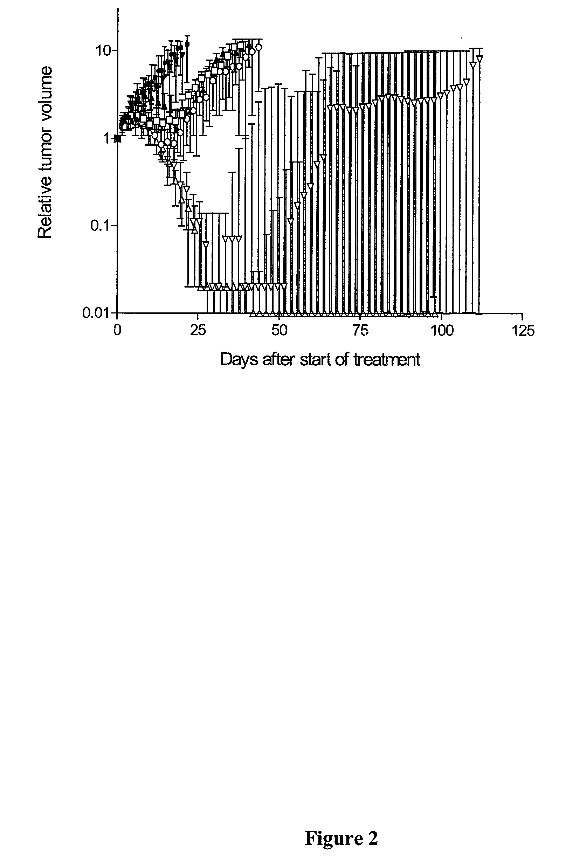

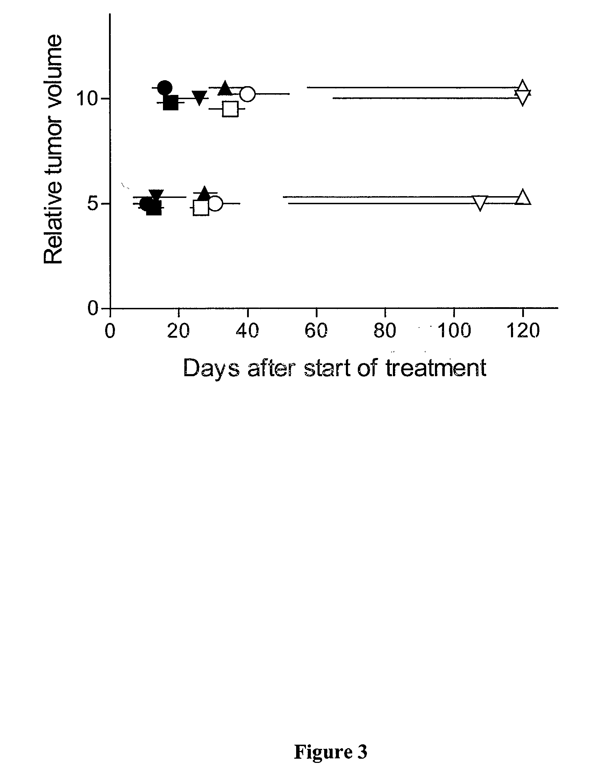

**BIWI 1 in combination with fractionated irradiation:** In FaDu tumors BIWA 4 did not alter tumor growth compared with control tumors treated with PBS (p-values 0.40-0.67, Table 1, Figure 2 and 3). BIWI 1 at a dose of 50 µg DM1/kg b.w./d did not prolong tᵥ₅ compared with controls (p= 0.54), while Tᵥ₁₀ was slightly but significant longer (p 0.025). Tᵥ₅ and Tᵥ₁₀ for FaDu tumors treated with BIWI 1 at a dose of 100 µg DM1/kg b.w./d were substantially and significantly prolonged compared to controls (p<0.0001).

Irradiation with 20 Gy in 5 fractions combined with PBS (irradiation control group) resulted in a significant prolongation of growth delay of about 2 weeks compared to unirradiated controls (p< 0.0001). No permanent local tumor controls were observed in this group. A slightly but significant longer growth delay was observed after application of BIWA 4 in combination with irradiation compared with irradiated controls (p 0.03-0.05). BIWI 1 at a dose of 50 µg DM1/kg b.w./d resulted in a clear-cut longer tᵥ₅ (p<0.0001) and tᵥ₁₀ (p<0.0001) compared with irradiated tumors which received PBS or BIWA 4. In the BIWI 1 (50 µg DM1/ kg/ d)-group 7 out of 15 tumours were locally controlled at day 120. BIWI 1 at a dose of 100 µg DM1/kg b.w./d resulted in a significant longer tᵥ₅ (p<0.0001) and tᵥ₁₀ (p<0.0001) compared with irradiated tumors which received PBS or BIWA 4. In the BIWI 1 (100 µg DM1/ kg/ d)-group 6 out of 15 tumours were locally controlled at day 120 day, additional 4 animals were censored without tumour after 56, 68, 104 and 110 days. Growth delay after irradiation combined with BIWI 1 (100 µg DM1/ kg/ d) was longer than after irradiation plus BIWI 1 (50 µg DM1/ kg/ d), however, this effect was not significant (p=0.89 for tᵥ₅; p=0.41 for tᵥ₁₀).

Taken together, BIWI 1 was well tolerated by NMRI nude mice. The study shows that, when given alone, 5 x BIWI 1 (100µg DM1/ kg/ d) significantly prolongs growth delay of FaDu tumors, while 5 x BIWI 1 (50µg DM1/ kg/ d) shows no or only little antitumor effect. Tumor growth delay after 5 x BIWI 1 (100 µg DM1/ kg/ d) was almost identical to tumor growth delay after irradiation with 5 x 4 Gy. From this observation and results of previous experiments is can be estimated that 5 x BIWI 1 (100 µg DM1/ kg/ d) kills at least two logs of clonogenic FaDu cells. BIWA 4 antibody control did not show any effect on FaDu tumors when given alone. When combined with fractionated irradiation, BIWA 4 enhanced the efficacy of treatment slightly but significantly (estimated dose modifying factor= 1.2). Despite of the fact that BIWI 1 (50 µg DM1/ kg/ d) alone showed little antitumor efficacy, growth delay after fractionated irraditation was greatly enhanced when this conjugated antibody was applied (estimated dose modifying factor= 4.1). This result clearly demonstrates an important radiosensitizing efficacy of BIWI 1(50 µg DM1/ kg/ d) and that this effect is supraadditive. The efficacy of the combined treatment was further increased when BIWI 1 (100 µg DM1/ kg/ d) was applied (estimated dose modifying factor > 4.5). Local tumor control rates at day 120 were 0/15 after fractionated irradiation alone, 0/14 after irradiation and BIWA 4, 7/15 after irradiation and BIWI 1 (50 µg DM1/kg/ d) and 6/15 plus 4 locally controlled animals censored at day 56, 68, 104, 110 after irradiation combined with BIWI 1 (100 µg DM1/ kg/ d). The observation of permanent local control shows that BIWI 1 can improve the curative potential of radiotherapy.

Therefore, it can be concluded that the cytotoxic radioimmunoconjugate of the invention, in particular BIWI 1 is a potent radiosensitizer . The effects of combined treatment are unexpectedly supraadditive. The observation of locally controlled tumors suggests that the cytotoxic immunoconjugates of the invention, in particular BIWI 1, can improve the curative potential of radiotherapy.

**Table 1:**

| Growth delay to 5 times (tᵥ₅) and 10 times (tᵥ₁₀) the starting volume for human FaDu SCC in the different experimental arms. d= days. fRT= fractionated irradiation | | |
|---|---|---|
| Experimental arm | tᵥ₅ (95% CI) | tᵥ₁₀ (95% CI) |
| PBS | 12.8 d (8; 16 d) | 17.8 d (14; 22 d) |
| BIWA 4 | 10,8 d (7; 13 d) | 16.0 d (12; 18 d) |
| BIWI 1 (50µg DM1) | 13.5 d (7; 22 d) | 26.0 d (16; 29 d) |
| BIWI 1 (100µg DM1) | 27.5 d (24; 31 d) | 33.5 d (29; 39 d) |
| PBS + fRT | 26.5 d (23; 28 d) | 35 d (29; 39 d) |
| BIWA 4+ fRT | 30.5 d (25; 38 d) | 40.0 d (35; 52 d) |
| BIWI 1 (50µg DM1)+ fRT | 107.5 d (52; >120 d) | >120 d (65; >120 d) |
| BIWI 1 (100µg DM1)+ fRT | >120 d (50; >120 d) | >120 d (57; >120 d) |

### Figures

**Figure 1:** Median relative body weight of the animals in the different experimental arms. Upper panel: drug treatment only. Lower panel: drug treatment combined with fractionated irradiation (5x4 Gy). Box: PBS, circle: BIWA 4, triangle downwards: BIWI 1 (50 µg DM1/kg/ d), triangle upwards: BIWI 1 (100 µg DM1/ kg/ d). Error bars represent 95% CI.

**Figure 2:** Growth curves of FaDu tumors in the different experimental arms. Closed symbols: drug treatment only. Open symbol: drug treatment combined with fractionated irradiation (5 x 4 Gy). Box: PBS, circle: BIWA 4, triangle downwards: BIWI 1 (50 µg DM1/ kg/ d), triangle upwards: BIWI 1 (100 µg DM1/ kg/ d). Error bars represent 95% CI.

**Figure 3:** Growth delay to 5 times and 10 times the relative tumor volume at start of treatment in the different experimental arms. Closed symbols: drug treatment only. Open symbol: drug treatment combined with fractionated irradiation (5 x 4 Gy). Box: PBS, circle: BIWA 4, triangle downwards: BIWI 1 (50 µg DM1/ kg/ d), triangle upwards: BIWI 1 (100 µg DM1/ kg/ d). Error bars represent 95% CI.

## Claims

1. Use of a compound of formula
**A(LB)**_{**n**}
wherein
**A** is an antibody molecule which is specific for CD44;
**L** is a linker moiety;
**B** is a compound which is toxic to cells; and
**n** is a decimal number with n = 1 to 10
for the preparation of a pharmaceutical composition for the treatment of cancer, wherein said compound is used or is for use in combination with radiotherapy.

2. The use of claim 1 wherein said linker moiety has a chemical bond capable of being cleaved inside a cell.

3. The use of claim 2 wherein said chemical bond is a disulfide bond.

4. The use of claims 1 to 3, wherein the antibody molecule is specific for the exon v6 of human CD44.

5. The use of claims 1 to 4, wherein the antibody molecule is specific for an epitope within the amino acid sequence SEQ ID NO: 3.

6. The use of claims 1 to 5, wherein the antibody molecule is the monoclonal antibody VFF-18 (DSM ACC2174) or a recombinant antibody having the complementary determining regions (CDRs) of VFF-18.

7. The use of claims 1 to 6, wherein the antibody molecule comprises light chains having the amino acid sequence SEQ ID NO: 4, or SEQ ID NO: 8, and heavy chains having the amino acid sequence SEQ ID NO: 6.

8. The use of claims 1 to 7, wherein the toxic compound B is a maytansinoid.

9. The use of claim 8 wherein the maytansinoid has the formula 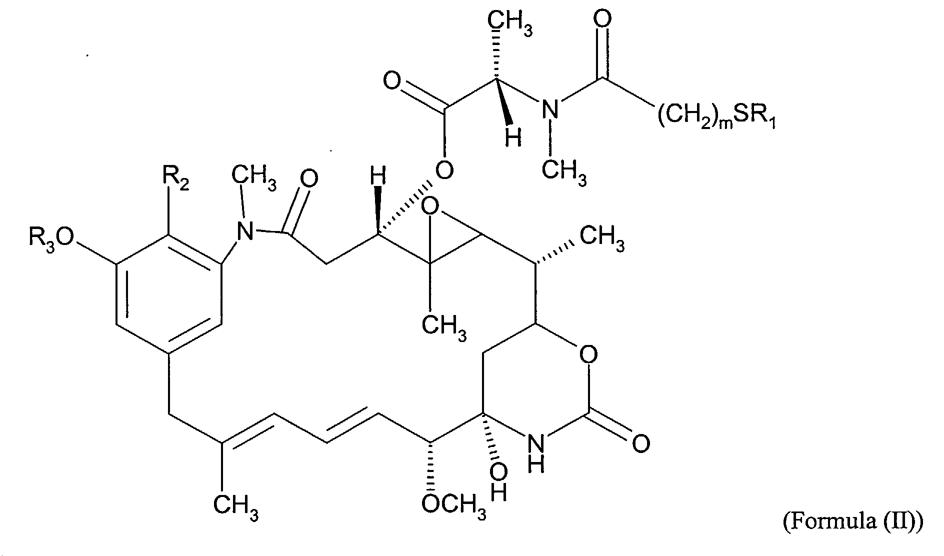 wherein
R₁ represents H or SR₄, wherein R₄ represents methyl, ethyl, linear alkyl, branched alkyl, cyclic alkyl, simple or substituted aryl, or heterocyclic;
R₂ represents Cl or H;
R₃ represents H or CH₃; and
m represents 1,2, or 3.

10. The use of claim 9, wherein R₁ is H or CH₃, R₂ is Cl, R₃ is CH₃, and m = 2

11. The use of claims 1 to 10, wherein the compound **A(LB)**_{**n**} is of formula 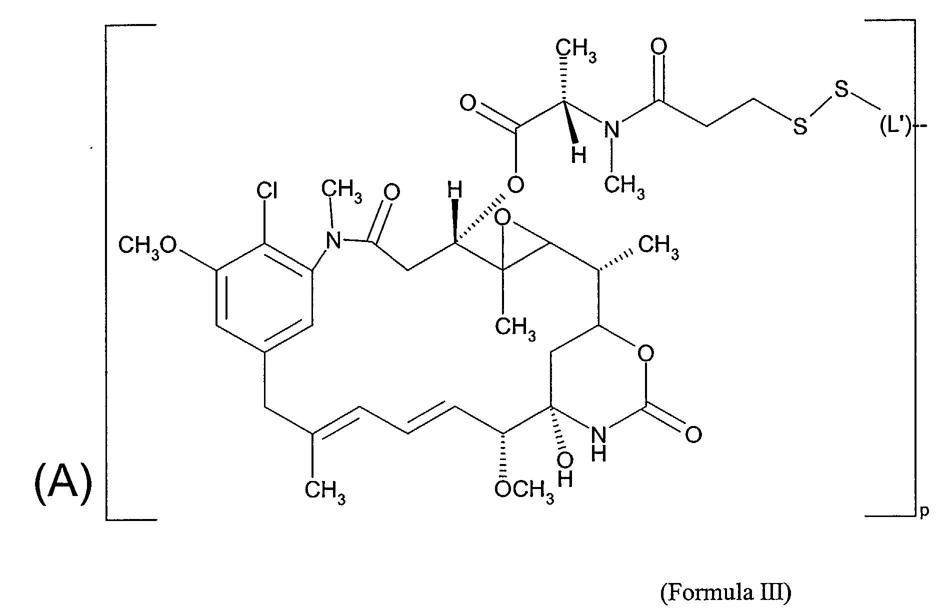 wherein
A is an antibody molecule which is specific for CD44.
(L') is an optional linker moiety
p is a decimal number with p = 1 to 10n

12. The use of claims 1 to 11 wherein p is 3 to 4.

13. The use of any one of claims 1 to 12, wherein the radiotherapy is external beam radiotherapy or radioimmunotherapy.

14. The use of claim 13, wherein the radiotherapy is fractionated radiotherapy.

15. The use of any one of claims 13 or 14, wherein the radioimmunotherapy comprises a radiolabeled antibody molecule which is specific for CD44.

16. The use of claim 15, wherein the radiolabeled antibody molecule comprises light chains having the amino acid sequence SEQ ID NO: 4, or SEQ ID NO: 8, and heavy chains having the amino acid sequence SEQ ID NO: 6.

17. Use of a conjugate of a CD44v6 specific antibody molecule and a maytansinoid for the manufacture of a pharmaceutical composition for the treatment of cancer, wherein said conjugate is used or is for use in combination with radiotherapy.

18. The use of of claim 17, wherein the antibody molecule is specific for an epitope within the amino acid sequence SEQ ID NO: 3.

19. The use of claim 18, wherein the antibody molecule is the monoclonal antibody VFF-18 (DSM ACC2174) or a recombinant antibody having the complementary determining regions (CDRs) ofVFF-18.

20. The use of of any one of claims 17 to 19, wherein said antibody molecule comprises light chains having the amino acid sequence SEQ ID NO: 4, or SEQ ID NO: 8, and heavy chains having the amino acid sequence SEQ ID NO: 6.

21. The use of any one of claims 17 to 20, wherein the maytansinoid is linked to the antibody molecule by a disulfide moiety.

22. The use of any one of claims 17 to 21, wherein the maytansinoid has the formula

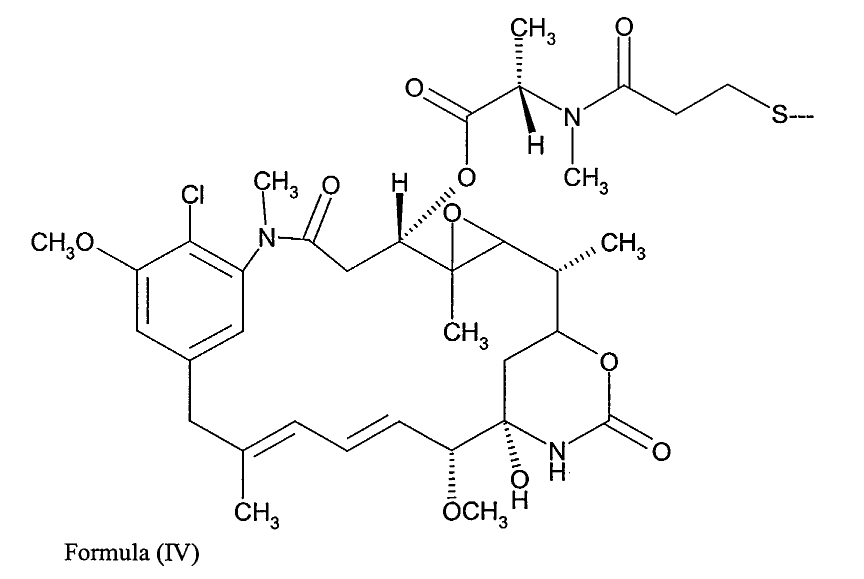

23. The use of a conjugate of a CD44v6 specific antibody molecule and a maytansinoid for the manufacture of a medicament for the treatment of cancer, wherein said compound is used or is for use in combination with radiotherapy, and wherein the antibody comprises light chains having the amino acid sequence SEQ ID NO: 4, and heavy chains having the amino acid sequence SEQ ID NO: 6, and wherein the maytansinoid has the formula 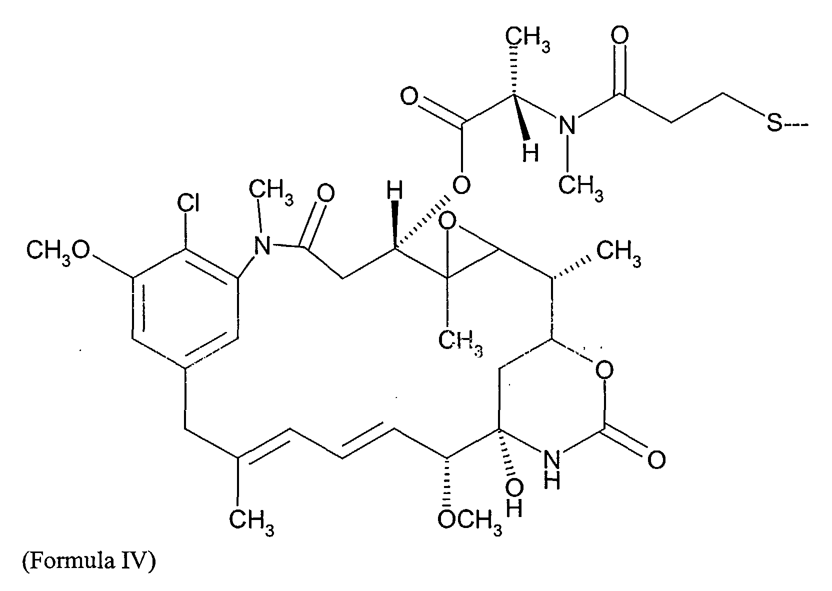 and is linked to the antibody through a disulfide bond.

24. The use of any one of claims 17 to 23, wherein one or more maytansinoid residues are linked to an antibody molecule.

25. The use of claim 24, wherein 3 to 4 maytansinoid residues are linked to an antibody molecule.

26. The use of any one of claims 17 to 25, wherein the maytansinoid is linked to the antibody molecule through a ―S-CH₂CH₂-CO-, a -S-CH₂CH₂CH₂CH₂-CO-, or a -S-CH(CH₃)CH₂CH₂-CO- group.

27. The use of any one of claims 17 to 26, wherein the radiotherapy in external beam radiotherapy or radioimmunotherapy.

28. The use of claim 27, wherein the radiotherapy is fractionated radiotherapy.

29. The use of claim 27 or 28 wherein the radioimmunotherapy comprises a radiolabeled antibody molecule which is specific for CD44.

30. The use of any one of claims 1 to 29, wherein the cancer is head and neck squameous cell carcinoma, esophagus squameous cell carcinoma, lung squameous cell carcinoma, skin squameous cell carcinoma, cervix squameous cell carcinoma, breast adenocarcinoma, lung adenocarcinoma, pancreas adenocarcinoma, colon adenocarcinoma, or stomach adenocarcinoma.

31. The use of any one of claims 13 to 16 or 27 to 30, wherein said compound **A(LB)**_{**n**} or conjugate, and the radio immunotherapeutic agent are formulated in separate pharmaceutical compositions.

32. The use of any one of claims 13 to 16 or 27 to 30, wherein said compound **A(LB)**_{**n**} or conjugate and the radioimmunotherapeutic agent are formulated in one single pharmaceutical composition.

33. Method of treatment of cancer in a patient in need thereof, comprising administering to the patient a therapeutically effective amount of a compound **A(LB)**_{**n**} as defined in any one of claims 1 to 12, or a conjugate as defined in any one of claims 17 to 26, in combination with radiotherapy as defined in any one of claims 13 to 16, or 27 to 29.

34. The method of claim 33, wherein the cancer is head and neck squameous cell carcinoma, esophagus squameous cell carcinoma, lung squameous cell carcinoma, skin squameous cell carcinoma, cervix squameous cell carcinoma, breast adenocarcinoma, lung adenocarcinoma, pancreas adenocarcinoma, colon adenocarcinoma, or stomach adenocarcinoma.

35. The method of claim 33 or 34, wherein the compound **A(LB)**_{**n**} or conjugate, and a radioimmunotherapeutic agent are administered separately.

36. The method of claim 33 or 34, wherein the compound **A(LB)**_{**n**} or conjugate, and the chemotherapeutic agent are administered as components of a single pharmaceutical composition.

37. Pharmaceutical composition comprising a compound **A(LB)**_{**n**} as defined in any one of claims or according to claims 1 to 12, or a conjugate as defined in any one of claims 17 to 26, together with a radioimmunotherapeutic agent as defined in any one of claims 13 to 16, or 27 to 29, and optionally further comprising one or more pharmaceutically acceptable carrier(s), diluent(s), or excipient(s).

38. A kit comprising, in separate pharmaceutical compositions, a compound **A(LB)**_{**n**} as defined in any one of claims 1 to 12, or a conjugate as defined in any one of claims 17 to 26, and a radioimmunotherapeutic agent as defined in any one of claims 13 to 16, or 27 to 29.

39. Use of a radioimmunotherapeutic agent for the preparation of a pharmaceutical composition for the treatment of cancer, wherein said radioimmunotherapeutic agent is used or is for use in combination with a compound of Formula **A(LB)**_{**n**} as defined in any of the preceding claims.

40. Use of a radioimmunotherapeutic agent for the preparation of a pharmaceutical composition for the treatment of cancer, wherein said chemotherapeutic agent is used or is for use in combination with a conjugate as defined in any one of claims 17 to 26.

41. The use of claim 39 or 40, wherein the radioimmunotherapeutic agent is radiolabeled CD44 specific antibody molecule.

42. The use of claim 41, wherein the radiolabeled antibody molecule comprises light chains having the amino acid sequence SEQ ID NO: 4, or SEQ ID NO: 8, and heavy chains having the amino acid sequence SEQ ID NO: 6.


SEQUENCE LISTING

<110> Boehringer Ingelheim International GmbH

<120> Cytotoxic CD44 Antibody Immunoconjugates

<130>

<140>
<141>

<160> 7

<170> PatentIn Ver. 2.1

<210> 1
<211> 42
<212> PRT
<213> Homo sapiens

<223> Description of Artificial Sequence: Human CD44 Exon v6 SEQ ID
NO: 1

<400> 1
Gln Ala Thr Pro Ser Ser Thr Thr Glu Glu Thr Ala Thr Gln Lys Glu
  1               5                  10                  15

Gln Trp Phe Gly Asn Arg Trp His Glu Gly Tyr Arg Gln Thr Pro Arg
             20                  25                  30

Glu Asp Ser His Ser Thr Thr Gly Thr Ala
         35                  40

<210> 2
<211> 14
<212> PRT
<213> Homo sapiens

<223> Description of Artificial Sequence: SEQ ID NO: 2

<400> 2
Gln Trp Phe Gly Asn Arg Trp His Glu Gly Tyr Arg Gln Thr
  1               5                  10

<210> 3
<211> 11
<212> PRT
<213> Homo sapiens

<223> Description of Artificial Sequence: SEQ ID NO: 3

<400> 3
Trp Phe Gly Asn Arg Trp His Glu Gly Tyr Arg
  1               5                  10

<210> 4
<211> 213
<212> PRT
<213> Artificial Sequence

<220>
<223> Description of Artificial Sequence: Humanised
      Murine Antibody BIWA 4 Light Chain SEQ ID NO: 4

<400> 4
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Ile Asn Tyr Ile
            20                  25                  30

Tyr Trp Tyr Gln Gln Lys Pro Gly Gln Ala Pro Arg Leu Leu Ile Tyr
        35                  40                  45

Leu Thr Ser Asn Leu Ala Ser Gly Val Pro Ala Arg Phe Ser Gly Ser
    50                  55                  60

Gly Ser Gly Thr Asp Phe Thr Leu Thr Ile Ser Ser Leu Glu Pro Glu
65                  70                  75                  80

Asp Phe Ala Val Tyr Tyr Cys Leu Gln Trp Ser Ser Asn Pro Leu Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210


<210> 5
<211> 702
<212> DNA
<213> Artificial Sequence

<220>
<223> Description of Artificial Sequence: Humanised
      Murine Antibody BIWA 4 Light Chain SEQ ID NO: 5

<400> 5
atggaagccc cagctcagct tctcttcctc ctgctgctct ggctcccaga taccaccgga 60
gaaattgttc tcacccagtc tccagcaacc ctgtctctgt ctccagggga gagggccacc 120
ctgtcctgca gtgccagctc aagtataaat tacatatact ggtaccagca gaagccagga 180
caggctccta gactcttgat ttatctcaca tccaacctgg cttctggagt ccctgcgcgc 240
ttcagtggca gtgggtctgg aaccgacttc actctcacaa tcagcagcct ggagcctgaa 300
gattttgccg tttattactg cctgcagtgg agtagtaacc cgctcacatt cggtggtggg 360
accaaggtgg agattaaacg tacggtggct gcaccatctg tcttcatctt cccgccatct 420
gatgagcagt tgaaatctgg aactgcctct gttgtgtgcc tgctgaataa cttctatccc 480
agagaggcca aagtacagtg gaaggtggat aacgccctcc aatcgggtaa ctcccaggag 540
agtgtcacag agcaggacag caaggacagc acctacagcc tcagcagcac cctgacgctg 600
agcaaagcag actacgagaa acacaaagtc tacgcctgcg aagtcaccca tcagggcctg 660
agctcgcccg tcacaaagag cttcaacagg ggagagtgtt ga                    702

<210> 6
<211> 444
<212> PRT
<213> Artificial Sequence

<220>
<223> Description of Artificial Sequence: Humanised
      Murine Antibody BIWA 4 Heavy Chain SEQ ID NO: 6

<400> 6
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Asp Met Ser Trp Val Arg Gln Ala Pro Gly Lys Gly Leu Glu Trp Val
        35                  40                  45

Ser Thr Ile Ser Ser Gly Gly Ser Tyr Thr Tyr Tyr Leu Asp Ser Ile
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Gln Gly Leu Asp Tyr Trp Gly Arg Gly Thr Leu Val Thr Val
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Lys Lys Val Glu Pro Lys Ser Cys Asp Lys Thr His Thr Cys Pro
    210                 215                 220

Pro Cys Pro Ala Pro Glu Leu Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Ala Leu Pro Ala Pro Ile Glu Lys Thr Ile Ser Lys Ala
                325                 330                 335

Lys Gly Gln Pro Arg Glu Pro Gln Val Tyr Thr Leu Pro Pro Ser Arg
            340                 345                 350

Asp Glu Leu Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Lys
        435                 440

<210> 7
<211> 1392

<212> DNA
<213> Artificial Sequence

<220>
<223> Description of Artificial Sequence: Humanised
      Murine Antibody BIWA 4 Heavy Chain SEQ ID NO: 7

<400> 7
atggagtttg ggctgagctg gctttttctt gtggctattt taaaaggtgt ccagtgtgaa 60
gtgcagctgg tggagtctgg gggaggctta gtgaagcctg gagggtccct aagactctcc 120
tgtgcagcct ctggattcac tttcagtagc tatgacatgt cttgggttcg ccaggctccg 180
gggaaggggc tggagtgggt ctcaaccatt agtagtggtg gtagttacac ctactatcta 240
gacagtataa agggccgatt caccatctcc agagacaatg ccaagaactc cctgtacctg 300
caaatgaaca gtctgagggc tgaggacacg gccgtgtatt actgtgcaag acaggggttg 360
gactactggg gtcgaggaac cttagtcacc gtctcctcag ctagcaccaa gggcccatcg 420
gtcttccccc tggcaccctc ctccaagagc acctctgggg gcacagcggc cctgggctgc 480
ctggtcaagg actacttccc cgaaccggtg acggtgtcgt ggaactcagg cgccctgacc 540
agcggcgtgc acaccttccc ggctgtccta cagtcctcag gactctactc cctcagcagc 600
gtggtgaccg tgccctccag cagcttgggc acccagacct acatctgcaa cgtgaatcac 660
aagcccagca acaccaaggt ggacaagaaa gttgagccca aatcttgtga caaaactcac 720
acatgcccac cgtgcccagc acctgaactc ctggggggac cgtcagtctt cctcttcccc 780
ccaaaaccca aggacaccct catgatctcc cggacccctg aggtcacatg cgtggtggtg 840
gacgtgagcc acgaagaccc tgaggtcaag ttcaactggt acgtggacgg cgtggaggtg 900
cataatgcca agacaaagcc gcgggaggag cagtacaaca gcacgtaccg tgtggtcagc 960
gtcctcaccg tcctgcacca ggactggctg aatggcaagg agtacaagtg caaggtctcc 1020
aacaaagccc tcccagcccc catcgagaaa accatctcca aagccaaagg gcagccccga 1080
gaaccacagg tgtacaccct gcccccatcc cgggatgagc tgaccaagaa ccaggtcagc 1140
ctgacctgcc tggtcaaagg cttctatccc agcgacatcg ccgtggagtg ggagagcaat 1200
gggcagccgg agaacaacta caagaccacg cctcccgtgc tggactccga cggctccttc 1260
ttcctctaca gcaagctcac cgtggacaag agcaggtggc agcaggggaa cgtcttctca 1320
tgctccgtga tgcatgaggc tctgcacaac cactacacgc agaagagcct ctccctgtct 1380
ccgggtaaat ga 1392

<210> 8
<211> 213
<212> PRT
<213> Artificial Sequence

<220>
<223> Description of Artificial Sequence: Humanised
      Murine Antibody BIWA 8 Light Chain SEQ ID NO: 8

<400> 8
Glu Ile Val Leu Thr Gln Ser Pro Ala Thr Leu Ser Leu Ser Pro Gly
1               5                   10                  15

Glu Arg Ala Thr Leu Ser Cys Ser Ala Ser Ser Ser Ile Asn Tyr Ile
            20                  25                  30

Tyr Trp Leu Gln Gln Lys Pro Gly Gln Ala Pro Arg Ile Leu Ile Tyr
        35                  40                  45

Leu Thr Ser Asn Leu Ala Ser Gly Val Pro Ala Arg Phe Ser Gly Ser
    50                  55                  60

Gly Ser Gly Thr Asp Phe Thr Leu Thr Ile Ser Ser Leu Glu Pro Glu

65                  70                  75                  80

Asp Phe Ala Val Tyr Tyr Cys Leu Gln Trp Ser Ser Asn Pro Leu Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210> 9
<211> 702
<212> DNA
<213> Artificial Sequence

<220>
<223> Description of Artificial Sequence: Humanised
      Murine Antibody BIWA 8 Light Chain SEQ ID NO: 9

<400> 9
atggaagccc cagctcagct tctcttcctc ctgctgctct ggctcccaga taccaccgga 60
gaaattgttc tcacccagtc tccagcaacc ctgtctctgt ctccagggga gagggccacc 120
ctgtcctgca gtgccagctc aagtataaat tacatatact ggctccagca gaagccagga 180
caggctccta gaatcttgat ttatctcaca tccaacctgg cttctggagt ccctgcgcgc 240
ttcagtggca gtgggtctgg aaccgacttc actctcacaa tcagcagcct ggagcctgaa 300
gattttgccg tttattactg cctgcagtgg agtagtaacc cgctcacatt cggtggtggg 360
accaaggtgg agattaaacg tacggtggct gcaccatctg tcttcatctt cccgccatct 420
gatgagcagt tgaaatctgg aactgcctct gttgtgtgcc tgctgaataa cttctatccc 480
agagaggcca aagtacagtg gaaggtggat aacgccctcc aatcgggtaa ctcccaggag 540
agtgtcacag agcaggacag caaggacagc acctacagcc tcagcagcac cctgacgctg 600
agcaaagcag actacgagaa acacaaagtc tacgcctgcg aagtcaccca tcagggcctg 660
agctcgcccg tcacaaagag cttcaacagg ggagagtgtt ga                    702